(19) Europäisches Patentamt
European Patent Office
Office européen des brevets

(11) Publication number: 0 395 251 A1

(12) **EUROPEAN PATENT APPLICATION**

(21) Application number: 90303806.5

(22) Date of filing: 09.04.90

(51) Int. Cl.[5]: C07D 417/12, C07D 413/12, C07D 403/12, C07D 491/04, C07D 279/16, C07D 265/36, C07D 241/42, A01N 47/36

(30) Priority: 12.04.89 AU 3651/89

(43) Date of publication of application: 31.10.90 Bulletin 90/44

(84) Designated Contracting States: AT BE CH DE DK ES FR GB GR IT LI LU NL SE

(71) Applicant: ICI AUSTRALIA LIMITED
1 Nicholson Street
Melbourne Victoria 3001(AU)

(72) Inventor: Bell, Stephen
18 Mirams Street
Ascot Vale 3032 Victoria(AU)
Inventor: Drygala, Peter
F205/1 Dick Ward Drive
Fannie Bay 0820 Northern Territory(AU)

(74) Representative: Hardman, Carol Pauline et al
Imperial Chemical Industries PLC Legal Department: Patents PO Box 6 Bessemer Road
Welwyn Garden City Herts, AL7 1HD(GB)

(54) **Herbicidal sulfonamides.**

(57) Compounds of the formula

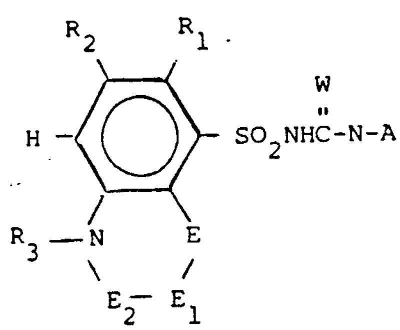

and salts thereof, W being O or S; a being a nitogen-containing heterocyclic ring system; E being O, $S(O)_m$ or $NR_4$ where m is 0-2; $E_1$ and $E_2$ being independently $CH_2$, $CH_2CH_2$, $CH(C_1\text{-}C_4$ alkyl), $C(C_1\text{-}C_4$ alkyl$)_2$ or CH(aryl), with the proviso that $E_1$ and $E_2$ cannot both be $CH_2CH_2$; $R_1$ and $R_2$ are independently hydrogen, halogen or one of a variety of organic substituents.
The compounds are effective herbicides.

EP 0 395 251 A1

## HERBICIDAL SULFONAMIDES

This invention relates to organic compounds having biological activity and in particular to organic compounds having herbicidal properties and plant growth regulating properties, to processes for the preparation of such compounds, to intermediates useful in the preparation of such compounds and to herbicidal compositions and processes utilizing such compounds.

The use of certain sulfonylurea derivatives as herbicides is known in the art. Thus, for example, the "Pesticide Manual" (C R Worthing Editor, The British Crop Protection Council, 7th Edition 1983) describes the sulfonylurea derivative known commercially as chlorsulfuron [1-(2-chlorophenylsulfonyl)-3-(4-methoxy-6-methyl-1,3,5-triazin--2-yl) urea] and its use as a broadleaf weed herbicide in cereals.

Australian Patent Application No. 23771/88 describes certain bicyclic sulfonyl urea herbicides and also cites a number of other bicyclic sulfonyl urea patents.

### Summary of the Invention

It has now been found that a new group of bicyclic sulfonylurea derivatives exhibit particularly useful herbicidal activity in both pre-emergent and post-emergent application. They have particularly high post-emergent herbicidal activity, and some show selective herbicidal activity against broad-leaved and grass weeds in important crops such as maize, rice, wheat, barley, sugarbeet, cotton and soya.

Accordingly the invention provides compounds of the formula I

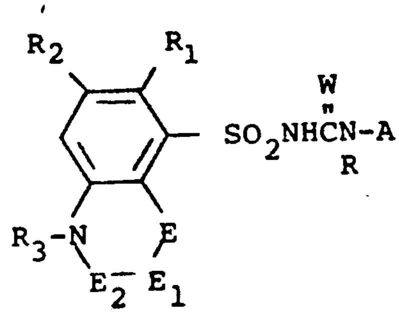

I

wherein

W is O or S;

E is O, $S(O)_m$, or $N-R_4$;

m is 0, 1 or 2;

$E_1$ and $E_2$ are independently

$CH_2$, $CH_2CH_2$, $CH(C_1-C_4$ alkyl), $C(C_1-C_4$ alkyl$)_2$ or CH (aryl), with the proviso that $E_1$ and $E_2$ cannot both be $CH_2CH_2$;

$R_1$ and $R_2$ are independently hydrogen, halogen, $C_1-C_4$ alkyl, $C_1-C_4$ haloalkyl, arylalkyl, nitro, $C_1-C_4$ alkoxy, $C_1-C_4$ haloalkoxy, cyano, carboxy, carbamoyl, N-($C_1-C_4$ alkyl)carbamoyl, N,N-di($C_1-C_4$ alkyl)carbamoyl, $C_1-C_4$ alkoxycarbonyl, $C_1-C_4$ alkylthio, $C_1-C_4$ alkylsulfinyl, $C_1-C_4$ alkylsulfonyl, sulfamoyl, $C_1-C_4$ alkylsulfamoyl, di($C_1-C_4$ alkyl) sulfamoyl, amino, $C_1-C_4$ alkylamino or di($C_1-C_4$ alkyl) amino, $C_1-C_4$ acylamino;

$R_1$ is selected from hydrogen, optionally substituted $C_1-C_4$ alkyl, $C_2-C_4$ alkenyl, $C_2-C_4$ alkynyl, or substituted aryl or arylalkyl;

$R_3$ and $R_4$ are each independently selected from hydrogen, optionally substituted $C_1-C_4$ alkyl, $C_2-C_4$ alkenyl, $C_2-C_4$ alkynyl, optionally substituted aryl or arylalkyl, or $C_1-C_4$ acyl;

A is 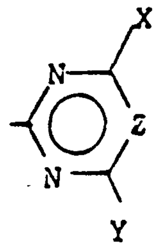 , 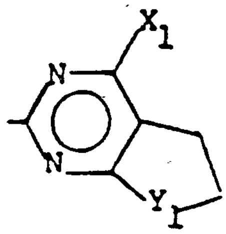 , 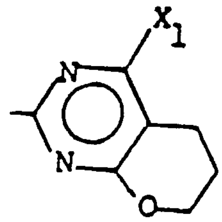 , 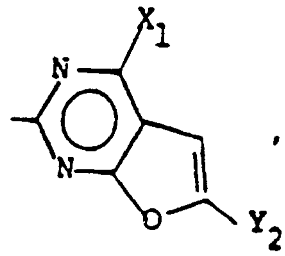 ,

A-1 A-2 A-3 A-4

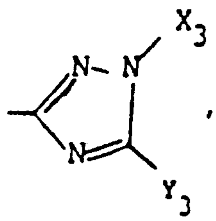 , 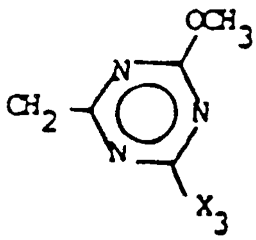 or 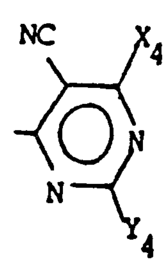

A-5 A-6 A-7

X is $CH_3$, $OCH_3$, $OCH_2CH_3$, Cl, F, Br, I, $OCF_2H$, $CH_2F$ or $CF_3$,
Y is H, $CH_3$, $OCH_3$, $OC_2H_5$, $CH_2OCH_3$, $NH_2$, $NHCH_3$, $N(CH_3)_2$, $C_2H_5$, $CF_3$, $SCH_3$, $N(OCH_3)CH_3$, $OCH_2CH{-}CH_2$, $OCH_2C{-}CH$, $OCH_2CF_3$, cyclopropyl, $OCH_2CH_2OCH_3$, $CH_2SCH_3$, $(C=O)R_5$, $CR_5(OCH_3)_2$,

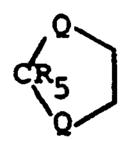

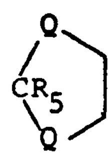 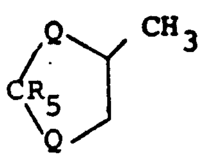 ,

$CR_5(OCH_2CH_3)_2$, $OCF_2H$, $SCF_2H$, C = CH or C = $CCH_3$
where Q is O or S and $R_5$ is H or $CH_3$; Z is CH, N, $CCH_3$, $CC_2H_5$, CCl or CBr; $Y_1$ is O or $CH_2$: $X_1$ is $CH_3$, $OCH_3$, $OC_2H_5$ or $OCF_2H$; $Y_2$ is H or $CH_3$; $X_2$ is $CH_3$, $C_2H_5$ or $CH_2CF_3$; $Y_3$ is $OCH_3$, $OC_2H_5$, $SCH_3$, $SC_2H_5$, $CH_3$ or $CH_2CH_3$; $X_3$ is $CH_3$ or $OCH_3$: $Y_4$ is $CH_3$, $OCH_3$, $OCH_2CH_3$ or Cl; and $X_4$ is $CH_3$, $OCH_3$, $OCH_2CH_3$, $CH_2OCH_3$ or Cl.

The invention also comprises the salts which the compounds of formula 1 are able to form with amines, alkali metal bases and alkaline earth metal bases, or with quaternary ammonium bases.

In the above definitions, the term "alkyl" used either alone or in compound words such as "alkylthio" or "haloalkyl", denotes straight chain or branched alkyl, e.g., methyl, ethyl, n-propyl, isopropyl or the different butyl isomers.

The term "aryl" used either alone or in the word "arylalkyl" denotes an aromatic ring such as phenyl or pyridyl, optionally substituted by halogen, methoxy, methyl, trifluoromethyl or nitro groups.

Alkoxy denotes methoxy, ethoxy, n-propoxy, isopropoxy and the different butyl isomers.
Alkenyl denotes straight chain or branched alkenes, e.g., vinyl, 1-propenyl, 2-propenyl, 3-propenyl or the different butenyl isomers.

Alkylsulfonyl denotes methylsulfonyl, ethylsulfonyl or the different propylsulfonyl isomers.
Alkylthio, alkylsulfinyl, alkylamino, etc. are defined in an analogous manner.

Preferred compounds of the invention include:

1) Those compounds of formula I where W is O and A is A-1.

2) Compounds of preferred 1) where R is hydrogen;
$R_1$ and $R_2$ are independently hydrogen, fluorine, chlorine, bromine, methyl, methoxy, trifluoromethyl, methoxycarbonyl, N,N-dimethylcarbamoyl, or methylthio; and
Y is methyl, methoxy, ethoxy, methoxymethyl, trifluoromethyl, 2,2,2-trifluoroethoxy, dimethoxymethyl, difluoromethoxy or methylamino.

A further group of preferred compounds of formula I comprises those compounds wherein :
E is O, S, NH or $NCH_3$;
$E_1$ is $CH_2$, $CH_2CH_2$ or $CH(CH_3)$;
$E_2$ is $CH_2$;
$R_3$ is hydrogen, methyl or ethyl.

Examples of the types of condensed saturated heterocyclic systems which are attached to the sulfonyl end of the sulfonylurea bridge are:
3,4-dihydro-2H-1,4-benzoxazines $B_1$,
3,4-dihydro-2H-1,4-benzothiazines $B_2$,
1,2,3,4-tetrahydroquinoxalines $B_3$,
2,3-dihydro-1,5-benzoxazepin $B_4$,
2,3-dihydro-1,5-benzothiazepin $B_5$, and
3,4-dihydro-1,5-benzodiazepine $B_6$.

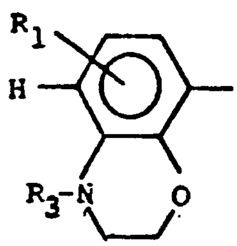 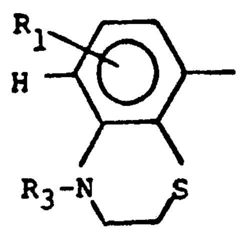 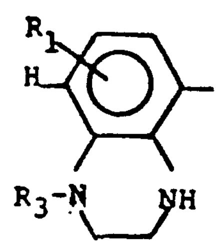

$B_1$ $B_2$ $B_3$

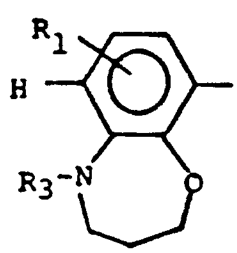 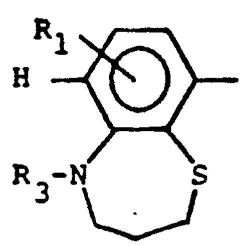 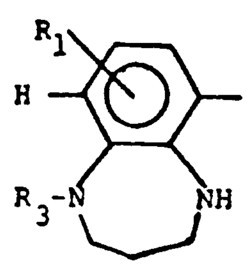

$B_4$ $B_5$ $B_6$

Specific examples of the compounds of the invention include those compounds listed in Table 1a and 1b.

TABLE 1a

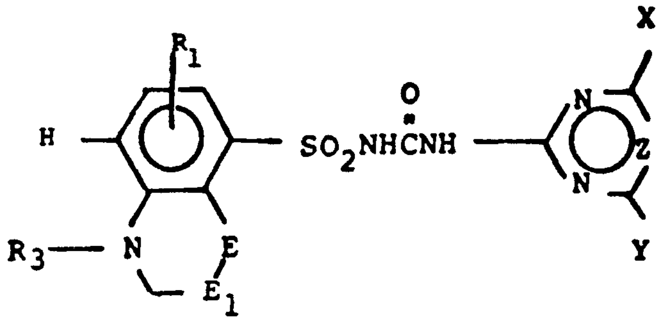

| Compound No. | $R^1$ | $R_3$ | E | $E_1$ | X | Y |
|---|---|---|---|---|---|---|
| 1 | H | H | O | $CH_2$ | $CH_3$ | $CH_3$ |
| 2 | H | H | O | $CH_2$ | $CH_3$ | $OCH_3$ |
| 3 | H | H | O | $CH_2$ | $OCH_3$ | $OCH_3$ |
| 4 | H | H | O | $CH_2$ | Cl | $OCH_3$ |
| 5 | H | H | O | $CH_2$ | $CH_3$ | $OCH_2CH_3$ |
| 6 | H | H | O | $CH_2$ | Cl | $NHCH_3$ |
| 7 | H | H | O | $CH_2$ | $OCF_2H$ | $OCF_2H$ |
| 8 | H | H | O | $CH_2$ | Cl | $N(CH_3)_2$ |
| 9 | H | H | O | $CH_2$ | Cl | $NH_2$ |
| 10 | H | H | O | $CH_2$ | $CH_3$ | $SCH_3$ |
| 11 | H | H | O | $CH_2$ | $CH_3$ | $CH_2OCH_3$ |
| 12 | H | H | O | $CH_2$ | $CF_3$ | $OCH_3$ |
| 13 | H | H | O | $CH_2$ | $OCH_3$ | $OCF_2H$ |
| 14 | H | H | O | $CH_2$ | $OCH_3$ | $N(CH_3)_2$ |
| 15 | H | H | O | $CHCH_3$ | $CH_3$ | $CH_3$ |
| 16 | H | H | O | $CHCH_3$ | $CH_3$ | $OCH_3$ |

Table 1a (Continued)

| Compound No. | $R^1$ | $R_3$ | E | $E_1$ | X | Y |
|---|---|---|---|---|---|---|
| 17 | H | H | O | $CHCH_3$ | $OCH_3$ | $OCH_3$ |
| 18 | H | $CH_3$ | O | $CH_2$ | $CH_3$ | $CH_3$ |
| 19 | H | $CH_3$ | O | $CH_2$ | $CH_3$ | $OCH_3$ |
| 20 | H | $CH_3$ | O | $CH_2$ | $OCH_3$ | $OCH_3$ |
| 21 | H | $CH_3$ | O | $CHCH_3$ | $CH_3$ | $CH_3$ |
| 22 | H | $CH_3$ | O | $CHCH_3$ | $CH_3$ | $OCH_3$ |
| 23 | H | $CH_3$ | O | $CHCH_3$ | $OCH_3$ | $OCH_3$ |
| 24 | 7-Cl | H | O | $CH_2$ | $CH_3$ | $OCH_3$ |
| 25 | 7-$CH_3$ | H | O | $CH_2$ | $CH_3$ | $OCH_3$ |
| 26 | 7-$OCH_3$ | H | O | $CH_2$ | $CH_3$ | $OCH_3$ |
| 27 | 7-$CO_2CH_3$ | H | O | $CH_2$ | $CH_3$ | $OCH_3$ |
| 28 | 7-$CON(CH_3)_2$ | H | O | $CH_2$ | $CH_3$ | $OCH_3$ |
| 29 | 6-Cl | H | S | $CH_2$ | $CH_3$ | $OCH_3$ |
| 30 | H | H | S | $CH_2$ | $CH_3$ | $OCH_3$ |
| 31 | H | H | S | $CH_2$ | $OCH_3$ | $OCH_3$ |
| 32 | H | H | S | $CHCH_3$ | $CH_3$ | $CH_3$ |
| 33 | H | H | S | $CHCH_3$ | $CH_3$ | $OCH_3$ |
| 34 | H | H | S | $CHCH_3$ | $OCH_3$ | $OCH_3$ |
| 35 | H | $CH_3$ | S | $CH_2$ | $CH_3$ | $CH_3$ |
| 36 | H | $CH_3$ | S | $CH_2$ | $CH_3$ | $OCH_3$ |
| 37 | H | $CH_3$ | S | $CH_2$ | $OCH_3$ | $OCH_3$ |
| 38 | H | $CH_3$ | S | $CHCH_3$ | $CH_3$ | $CH_3$ |
| 39 | H | $CH_3$ | S | $CHCH_3$ | $CH_3$ | $OCH_3$ |
| 40 | H | $CH_3$ | S | $CHCH_3$ | $OCH_3$ | $OCH_3$ |
| 41 | 6-Cl | H | S | $CHCH_3$ | $CH_3$ | $CH_3$ |
| 42 | 6-Cl | H | S | $CHCH_3$ | $CH_3$ | $OCH_3$ |
| 43 | 6-Cl | H | S | $CHCH_3$ | $OCH_3$ | $OCH_3$ |
| 44 | 6-Cl | $CH_3$ | S | $CHCH_3$ | $CH_3$ | $OCH_3$ |
| 45 | H | H | SO | $CH_2$ | $CH_3$ | $CH_3$ |
| 46 | H | H | SO | $CH_2$ | $CH_3$ | $OCH_3$ |
| 47 | H | H | SO | $CH_2$ | $OCH_3$ | $OCH_3$ |

Table 1a (Continued)

| Compound No. | $R^1$ | $R_3$ | E | $E_1$ | X | Y |
|---|---|---|---|---|---|---|
| 48 | H | H | SO | $CHCH_3$ | $CH_3$ | $CH_3$ |
| 49 | H | H | SO | $CHCH_3$ | $CH_3$ | $OCH_3$ |
| 50 | H | H | SO | $CHCH_3$ | $OCH_3$ | $OCH_3$ |
| 51 | H | $CH_3$ | SO | $CH_2$ | $CH_3$ | $CH_3$ |
| 52 | H | $CH_3$ | SO | $CH_2$ | $CH_3$ | $OCH_3$ |
| 53 | H | $CH_3$ | SO | $CH_2$ | $OCH_3$ | $OCH_3$ |
| 54 | H | $CH_3$ | SO | $CHCH_3$ | $CH_3$ | $CH_3$ |
| 55 | H | $CH_3$ | SO | $CHCH_3$ | $CH_3$ | $OCH_3$ |
| 56 | H | $CH_3$ | SO | $CHCH_3$ | $OCH_3$ | $OCH_3$ |
| 57 | 6-Cl | H | SO | $CH_2$ | $CH_3$ | $CH_3$ |
| 58 | 6-Cl | H | SO | $CH_2$ | $CH_3$ | $OCH_3$ |
| 59 | 6-Cl | H | SO | $CH_2$ | $OCH_3$ | $OCH_3$ |
| 60 | H | H | $SO_2$ | $CH_2$ | $CH_3$ | $CH_3$ |
| 61 | H | H | $SO_2$ | $CH_2$ | $CH_3$ | $OCH_3$ |
| 62 | H | H | $SO_2$ | $CH_2$ | $OCH_3$ | $OCH_3$ |
| 63 | H | H | $SO_2$ | $CHCH_3$ | $CH_3$ | $CH_3$ |
| 64 | H | H | $SO_2$ | $CHCH_3$ | $CH_3$ | $OCH_3$ |
| 65 | H | H | $SO_2$ | $CHCH_3$ | $OCH_3$ | $OCH_3$ |
| 66 | 7-Cl | H | $SO_2$ | $CH_2$ | $CH_3$ | $OCH_3$ |
| 67 | 7-OMe | H | $SO_2$ | $CH_2$ | $CH_3$ | $OCH_3$ |
| 68 | H | $CH_3$ | $SO_2$ | $CH_2$ | $CH_3$ | $OCH_3$ |
| 69 | H | $CH_3$ | $SO_2$ | $CHCH_3$ | $CH_3$ | $CH_3$ |
| 70 | H | $CH_3$ | $SO_2$ | $CHCH_3$ | $CH_3$ | $OCH_3$ |
| 71 | H | $CH_3$ | $SO_2$ | $CHCH_3$ | $OCH_3$ | $OCH_3$ |
| 72 | 6-Cl | H | $SO_2$ | $CH_2$ | $CH_3$ | $CH_3$ |
| 73 | 6-Cl | H | $SO_2$ | $CH_2$ | $CH_3$ | $OCH_3$ |
| 74 | 6-Cl | H | $SO_2$ | $CH_2$ | $OCH_3$ | $OCH_3$ |
| 75 | H | n-Bu | $SO_2$ | $CH_2$ | $CH_3$ | $CH_3$ |

Table 1a (Continued)

| Compound No. | $R^1$ | $R_3$ | E | $E_1$ | X | Y |
|---|---|---|---|---|---|---|
| 76 | H | n-Bu | $SO_2$ | $CH_2$ | $CH_3$ | $OCH_3$ |
| 77 | H | n-Bu | $SO_2$ | $CH_2$ | $OCH_3$ | $OCH_3$ |
| 78 | H | H | NH | $CH_2$ | $CH_3$ | $OCH_3$ |
| 79 | H | H | $NCH_3$ | $CH_2$ | $CH_3$ | $OCH_3$ |
| 80 | H | $CH_3$ | NH | $CH_2$ | $CH_3$ | $OCH_3$ |
| 81 | H | $CH_3$ | $NCH_3$ | $CH_2$ | $CH_3$ | $OCH_3$ |
| 82 | H | CHO | O | $CH_2$ | $OCH_3$ | $OCH_3$ |
| 83 | H | $CH_3CO$ | O | $CH_2$ | $OCH_3$ | $OCH_3$ |

Table 1b

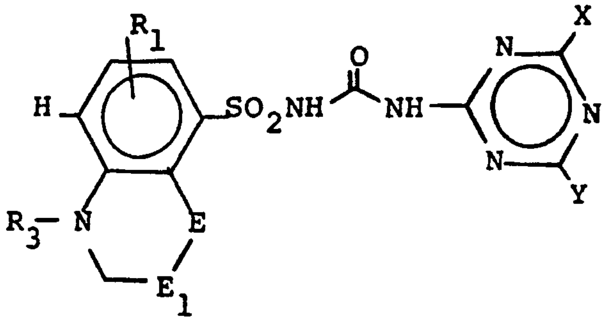

| Compound No. | $R^1$ | $R_3$ | E | $E_1$ | X | Y |
|---|---|---|---|---|---|---|
| 84 | H | H | O | $CH_2$ | $CH_3$ | $OCH_3$ |
| 85 | H | H | O | $CH_2$ | $OCH_3$ | $OCH_3$ |
| 86 | H | H | O | $CH_2$ | $OCH_2CH_3$ | $NHCH_3$ |
| 87 | H | H | O | $CHCH_3$ | $CH_3$ | $OCH_3$ |
| 88 | H | $CH_3$ | O | $CH_2$ | $CH_3$ | $OCH_3$ |
| 89 | H | $CH_3$ | O | $CHCH_3$ | $CH_3$ | $OCH_3$ |

Table 1b (Continued)

| Compound No. | $R^1$ | $R_3$ | E | $E_1$ | X | Y |
|---|---|---|---|---|---|---|
| 90 | 7-Cl | H | O | $CH_2$ | $CH_3$ | $OCH_3$ |
| 91 | $7-CO_2CH_3$ | H | O | $CH_2$ | $CH_3$ | $OCH_3$ |
| 92 | $7-CON(CH_3)_2$ | H | O | $CH_2$ | $CH_3$ | $OCH_3$ |
| 93 | H | H | S | $CH_2$ | $CH_3$ | $OCH_3$ |
| 94 | H | H | S | $CHCH_3$ | $CH_3$ | $OCH_3$ |
| 95 | H | $CH_3$ | S | $CH_2$ | $CH_3$ | $OCH_3$ |
| 96 | H | $CH_3$ | S | $CHCH_3$ | $CH_3$ | $OCH_3$ |
| 97 | H | H | SO | $CH_2$ | $CH_3$ | $OCH_3$ |
| 98 | H | H | SO | $CHCH_3$ | $CH_3$ | $OCH_3$ |
| 99 | H | $CH_3$ | SO | $CH_2$ | $CH_3$ | $OCH_3$ |
| 100 | H | $CH_3$ | SO | $CHCH_3$ | $CH_3$ | $OCH_3$ |
| 101 | 6-Cl | H | SO | $CH_2$ | $CH_3$ | $OCH_3$ |
| 102 | H | H | $SO_2$ | $CH_2$ | $CH_3$ | $OCH_3$ |
| 103 | H | H | $SO_2$ | $CHCH_3$ | $CH_3$ | $OCH_3$ |
| 104 | H | $CH_3$ | $SO_2$ | $CH_2$ | $CH_3$ | $OCH_3$ |
| 105 | H | $CH_3$ | $SO_2$ | $CHCH_3$ | $CH_3$ | $OCH_3$ |
| 106 | 6-Cl | H | $SO_2$ | $CH_2$ | $CH_3$ | $OCH_3$ |
| 107 | H | n-Bu | $SO_2$ | $CH_2$ | $CH_3$ | $OCH_3$ |
| 108 | H | H | $NCH_3$ | $CH_2$ | $CH_3$ | $OCH_3$ |
| 109 | H | $CH_3$ | $NCH_3$ | $CH_2$ | $CH_3$ | $OCH_3$ |

The compounds of the invention may be prepared by a variety of methods and in a further aspect the invention provides methods for the preparation of compounds of formula I.

Conveniently the preparation of the compounds of the invention can be considered in three parts.

Part A involves the preparation of fused phenylsulfonamides of the formula II

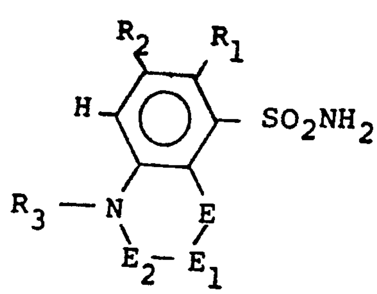

II

wherein $R_1$, $R_2$, $R_3$, E, $E_1$ and $E_2$ are as defined for formula I. The sulfonamides of formula II can be prepared in a variety of ways including the general methods outlined below.

The dihydro benzo-azine sulfonamides III can be prepared by reduction of the corresponding benzo-azinones IV with sodium borohydride and aluminium chloride by a modification of the procedure described by H.C. Brown and B.C. Subba Rao, J. Am Chem. Soc., 78, 2582 1956. Lithium aluminium hydride or sodium acyloxyborohydrides can also be used for this reduction.

Alternatively the substituted dihydro benzoazine ring system VI may in some instances be formed by direct alkylation of a suitable aniline derivative V.

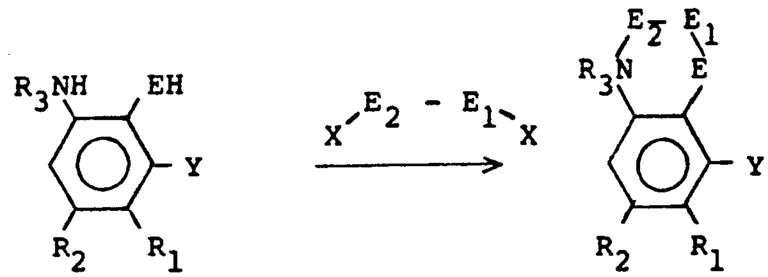


V VI

wherein E, $E_1$, $E_2$, $R_1$, $R_2$ and $R_3$ are as defined for formula I, X is a leaving group such as halogen and Y is a nitro or protected sulfamoyl group.

The preparation of aromatic sulfonamides from the corresponding anilines by diazotization and reaction of the diazonium salt with sulfur dioxide is well known in the art (J. Org; Chem;, 25, 1824 (1960)).

$$ArNH_2 \xrightarrow[2)\ SO_2,\ CuCl_2]{1)\ NaNO_2/HCl} ArSO_2Cl \xrightarrow{NH_3} ArSO_2NH_2$$

Some of the necessary amino-benzo-azinones have been described in the literature (see for example "Rodds Chemistry of Carbon Compounds", Vol. IV H, p 463, 1978) and they may be prepared for example by reduction of the corresponding nitro compounds.

Part B of the preparation of the compounds of the invention involves the preparation of the various nitrogen-containing heterocycles A-1 to A-7.

The heterocyclic amines of Formula VII can be prepared by methods known in the literature, or simple modifications thereof, by one skilled in the art.

H N -A
R

VII

For a review of the synthesis and reactions of 2- amino- and 2-methylaminopyrimidines (VII, A=A-1, Z = CH) see The Chemistry of Heterocyclic Compounds, Vol. 16, Wiley-Interscience, New York (1962). For a review of the synthesis and reactions of 2-amino- and 2-methylamino-s-triazines (VII, A=A-1, Z=N), see The Chemistry of Heterocyclic Compounds, Vol. 13, Wiley-Interscience, New York (1959), and F.C. Schaefer and K.R. Huffman, J. Org. Chem., 28, 1812 (1963). EP-A No. 84,224 and W. Braker et al., J. Amer. Chem. Soc., 69, 3072 (1947) describe methods for preparing aminopyrimidines and triazines substituted by acetal groups such as dialkoxymethyl or 1,3-dioxolan-2-yl. U.S. Patent 4,515,626 describes methods for the synthesis of cyclopropyl-pyrimidines and synthesis of cyclopropyl-pyrimidines and triazines substituted by such groups as alkyl, haloalkyl, alkoxy, haloalkoxy, alkylamino, dialkylamino or alkoxyalkyl.

The 5,6-dihydrofuro[2.3-d]pyrimidine-2-amines, the cyclopenta[d]pyrimidines-2-amines (VII, A is A-2) and the 6,7-dihydro-5N-pyrano[2,3-d]pyrimidin-2-amines (VII, A is A-3) can be prepared as taught in U.S. 4,339,267. The furo[2,3-d]pyrimidin-2-amines (VII, A is A-4) are described in U.S. 4,487,626.

Compounds of formula VII where A is A-5, are described in EP-A-0 073 562. Compounds of Formula I where A is A-6, are described in U.S. 4,496,392.

The amines of Formula VII where A is A-7 can be prepared by methods taught in EP-A-0 125 864 (published 21/11/84) or by suitable modifications that would be obvious to one skilled in the art.

Part C of the preparation of the compounds of the invention (formula I) involves the coupling of the sulfonamides of formula II with the heterocyclic amines of formula VII. The compounds of formula I can be prepared by one or more of the methods described below.

a) Many of the compounds of formula I can be prepared by reacting a sulfonylisocyanate or a sulfonylisothiocyanate of formula VIII with a heterocyclic amine of formula VII.

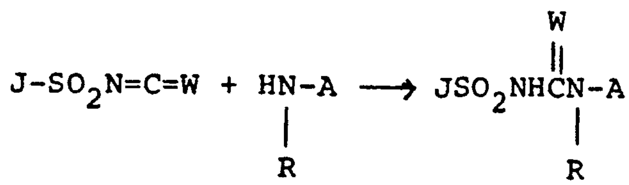

VIII VII I

where J represents the bicyclic system

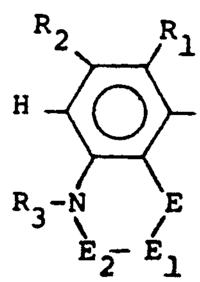

and W, A and R are as previously defined.

The reaction is carried out at 25° to 100°C in an inert, aprotic solvent such as methylene chloride or xylene for 0.5 to 24 hours as taught in U.S. Patent 4,127,405.

The intermediate sulfonylisocyanates (VIII, W=O) and isothiocyanates (VIII, W=S) are prepared by a variety of methods which are well known in the art and are described for example in EP-A-0 212 779 and the references cited therein.

b) Many of the compounds of formula I, where W is oxygen, can be prepared by reacting a phenyl carbamate of formula IX with a suitable amine of formula VII.

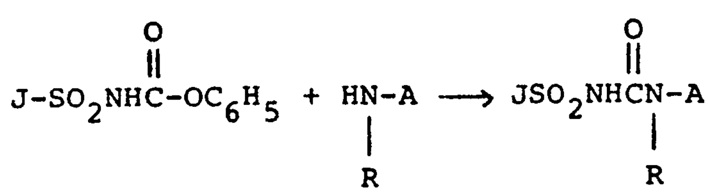

IX VII Ia

The reaction is carried out at 50° to 100°C in a solvent such as dioxane for 0.5 to 24 hours. The required carbamates IX are prepared by reacting the corresponding sulfonamides II with diphenylcarbonate in the presence of a strong base.

c) Compounds of formula Ia can also be made by reacting a heterocyclic carbamate of formula X with a suitable sulfonamide of formula II.

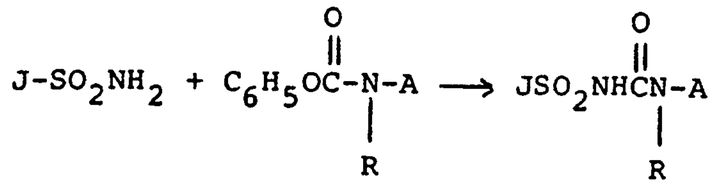

II X Ia

The reaction is carried out at 0° to 100°C in a solvent such as acetonitrile or dioxane or N,N-dimethylformamide in the presence of a non-nucleophilic base such as DBU for 0.2 to 24 hours. The required phenylcarbamates X are prepared by reacting the corresponding heterocyclic amines VII with diphenylcarbonate or phenylchloroformate in the presence of a strong base. Alternatively, the methylcarbamate derivative of the heterocyclic amine VII may be used.

d) Some of the compounds of the invention of formula Ib can be prepared by reacting a sulfonamide II with a heterocyclic isocyanate or isothiocyanate of formula XI.

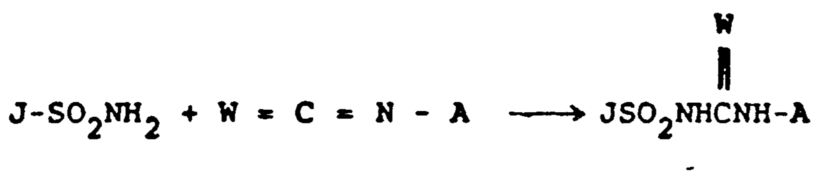

II XI Ib

The reaction is carried out at 25° to 80°C in an inert, aprotic solvent such as acetone or acetonitrile in the presence of a base such as potassium carbonate for 0.5 to 24 hours. The required heterocyclic isocyanates and isothiocyanates XI may be prepared from the corresponding amines $H_2NA$ which would be known to one skilled in the art as taught in EP-A-0 035 893.

e) Some of the compounds of formula I can be prepared by reacting sulfonamide (II) with a heterocyclic amide of formula (XII).

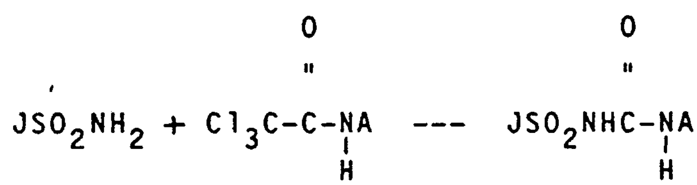

II XII Ic

The reaction is carried out with an appropriate base in a solvent at 50-120°C for 0.5 to 6 h.
The intermediate heterocyclic amides (XII) are prepared by a modification of an analogous procedure as described by N.M. Mollor et al Synthesis, 734 (1987).

In each of parts b), c), d) and e) above the groups J, W, A and R are as previously described.

The compounds of formula I wherein E is SO or $SO_2$ can be prepared in a variety of ways including from the appropriate sulfonamides of formula II wherein E is SO or $SO_2$, or from compounds of formula I wherein E is S by standard oxidation methods known in the literature.

Certain of the intermediate compounds of formulae III and VI are novel compounds and therefore in further embodiments the invention provides novel compounds of formulae III and VI and processes for the preparation thereof.

Agriculturally suitable salts of compounds of Formula I are also useful herbicides and can be prepared in a number of ways known to the art. For example, metal salts can be made by contacting compounds of Formula I with a solution of an alkali or alkaline earth metal salt having a sufficiently basic anion (e.g.

hydroxide, alkoxide, carbonate or hydroxide). Quaternary amine salts can be made by similar techniques.

Salts of compounds of Formula I can also be prepared by exchange by one cation to another. Cationic exchange can be effected by direct treatment of an aqueous solution of a salt of a compound of formula I, (e.g, alkali metal or quaternary amine salt) with a solution containing the cation to be exchanged. This method is most effective when the desired salt containing the exchanged cation is insoluble in water, e.g., a copper salt, and can be separated by filtration.

Exchange may also be effected by passing an aqueous solution of a salt of a compound of Formula I, (e.g., an alkali metal or quaternary amine salt) through a column packed with a cation exchange resin containing the cation to be exchanged. In this method, the cation of the resin is exchanged for that of the original salt and the desired product is eluted from the column. This method is particularly useful when the desired salt is water-soluble, eg., a potassium sodium or calcium salt.

Acid addition salts, useful in this invention, can be obtained by reacting a compound of Formula I, with a suitable acid, e.g., p-toluenesulfonic acid, trichloroacetic acid or the like.

The compounds of formula I are active as herbicides and therefore, in a further aspect the invention provides a process for severely damaging or killing unwanted plants which process comprises applying to the plants, or to the growth medium of the plants, an effective amount of a compound of formula I as hereinbefore defined.

The compounds of formula I may be applied directly to the plant (post-emergence application) or to the soil before the emergence of the plant (pre-emergence application)

The compounds of formula I may be used on their own to inhibit the growth of, severely damage, or kill plants but are preferably used in the form of a composition comprising a compound of the invention in admixture with a carrier comprising a solid or liquid diluent. Therefore, in yet a further aspect the invention provides growth inhibiting, plant damaging, or plant killing compositions comprising a compound of formula I as hereinabove defined and an inert carrier therefor.

Certain of the compounds of formula I exhibit useful plant growth regulating activity.

Plant growth regulating effects may be manifested in a number of ways. For example, suppression of apical dominance, stimulation of auxiliary bud growth, stimulating of early flowering and seed formation, enhancement of flowering and increase in seed yield, stem thickenings, stem shortening and tillering.

Accordingly in a still further aspect the invention provides a process for regulating the growth of a plant which process comprises applying to the plant, to the seed of the plant, or to the growth medium of the plant, an effective amount of a compound of formula I, as hereinbefore defined.

To effect the plant growth regulating process of the present invention the compounds of formula I may be applied directly to the plant (post-emergence application) or to the seed or soil before the emergence of the plant (pre-emergence) application.

The compounds of formula I may be used on their own to regulate the growth of plants but in general are preferably used in the form of a composition comprising a compound of the invention in admixture with a carier comprising a solid or liquid diluent. Therefore, in a still further aspect the invention provides plant growth regulating compositions comprising a compound of formula I as hereinbefore defined and an inert carrier therefor.

Compositions according to the invention include both dilute compositions, which are ready for immediate use, and concentrated compositions, which require to be diluted before use, usually with water. Preferably the compositions contain from 1 ppm to 2% of active ingredient, while concentrated compositions may contain from 20 to 99% of active ingredient, although from 20 to 70% is usually preferred.

The solid compositions may be in the form of granules, or dusting powders wherein the active ingredient is mixed with a finely divided solid diluent, eg kaolin, bentonite, kieselguhr, dolomite, calcium carbonate, talc, powdered magnesia, Fuller's earth and gypsum. They may also be in the form of dispersible powders or grains, comprising a wetting agent to facilitate the dispersion of the powder or grains in liquid. Solid compositions in the form of powders may be applied as foliar dusts.

Liquid compositions may comprise a solution or disperion or an active ingredient in water optionally containing a surface-active agent, or may comprise a solution or dispersion of an active ingredient in a water-immiscible organic solvent which is dispersed as droplets in water.

The rate of application of the compounds of the invention will depend on a number of factors including, for example, the compound chosen for use, the identity of the plants to be treated, the formulations selected for use and whether the compound is to be applied for foliage or root uptake. As a general guide, however, an application rate of from 0.001 to 10 kilograms, per hectare is suitable while from 0.01 to 5 kilograms per hectare may be preferred.

The compositions of the invention may comprise, in addition to one or more compounds of the invention, one or more compounds not of the invention but which possess biological activity. In certain

applications it may be preferred to use a herbicidal composition comprising a mixture of at least one herbicidal compound of formula I as hereinbefore defined and at least one other herbicide.

The compounds of this invention and their preparation are further illustrated by the following examples.

Example 1

Addition of thioglycollic acid to 1,4-dichloro-2,6-dinitrobenzene

Thioglycollic acid (12.28 ml, 0.18 mole) was added to a stirred suspension of sodium carbonate (41 g, 39 mole) and 1,4-dichloro-2,6-dinitrobenzene (40.0 g, 0.17 mole) in ethanol (1000 ml). The suspension was stirred at room temperature overnight and the resulting orange suspension diluted with ether (500 ml), acidified with conc. hydrochloric acid and the aqueous layer extracted with further ether (2 x 200 ml). The combined ether extracts were washed with saturated sodium chloride solution (2x), dried ($Na_2SO_4$) and concentrated to afford an orange solid of the thiophenoxy acetic acid (47.7 g, 97%). $\delta^1H(d_6$ -acetone) 8.2 (s, 2H); 3.8 (s, 2H).

Example 2

8-Amino-6-chloro-3,4-dihydro-2H-1,4-benzothiazin-3-one

Concentrated hydrochloric acid (450 ml) was added cautiously to an ice-bath cooled solution of the thiophenoxy acetic acid from Example 1 (32.6 g, 0.11 mole) and tin metal (powder, 90 g) in ethanol (60 ml). The initially orange reaction solution was then heated on a boiling steam bath for 2 h before cooling to room temperature. The resulting colourless solution was then poured slowly into a stirring ice-bath cooled solution of aqueous sodium hydroxide (300 g in 700ml) and stirring continued for 15 min. The resulting cloudy suspension was extracted into ethyl acetate (3 x 300 ml) and the combined extracts washed with saturated sodium chloride solution (3x). The organic layer was then filtered through a pad of celite, dried ($Na_2SO_4$) and concentrated to afford the amino-benzothiazinone as a colourless solid (18.6 g, 78%). $\delta^1H$ ($d_6$ -acetone) 9.45 (brs, 1H); 6.49 (d, J=2Hz, 1H); 6.43 (d, J=2Hz, 1H); 5.04 (brs, 2H); 3.39 (s, 2H).

Example 3

6-Chloro-8-sulfonamido-3,4-dihydro-2H-1,4-benzothiazin-3-one

A solution of sodium nitrite (7.23 g, 0.10 mole) in water (10 ml) was added over 10 min to a cooled (0°) suspension of the 8-amino benzothiazin-3-one (from Example 2) (18.0 g, 0.08 mole) in concentated hydrochloric acid (200 ml) and glacial acetic acid (37 ml). Following 30 mins stirring the golden-brown solution of the diazonium salt was added over 5 min to a stirred solution of cuprous chloride (3.0 g) in concentrated hydrochloric acid (100 ml) and acetic acid (100 ml) saturated with sulphur dioxide, all at 0°. The ice-bath was removed after 15 mins and the solution allowed to warm to room temperature and stir for a further 2 h. The resulting suspension was diluted to 1 litre with water, filtered and washed well with water to afford the sulfonyl chloride on drying as a pink-tan solid (22.2 g). $\delta^1H$ ($d_6$ -acetone/$d_6$ -DMSO) 11.10 (brs, 1H); 7.74 (d, J=2.2Hz, 1H); 7.60 (d, J=2.2Hz, 1Hz); 3.65 (s, 2H).

Concentrated aqueous ammonia (300 ml) was added over 5 min to a vigorously stirred suspension of the sulphonyl chloride in ether (200 ml). The solution obtained was heated on a boiling steam bath for 2 h, cooled, filtered and the tan solid was washed with cold water and dried to afford a pink-tan solid of the sulfonamide (16.6 g, 71%). $\delta^1H(d_6$ -DMSO) 10.98 (s, 1H); 7.66 (s, 2H); 7.52 (d, J=2.2Hz, 1H); 7.20 (d, J=2.2Hz, 1H); 3.58 (s, 2H).

Example 4

6-Chloro-8-sulfonamido-3,4-dihydro-2H-1,4-benzothiazine

A suspension of sodium borohydride (3.39 g, 0.09 mole) and aluminium chloride (3.99 g, 0.03 mole) in dimethoxy ethane (200 ml) was stirred at room temperature for 30 min. The sulfonamidobenzothiazinone (5.0 g, 0.02 mole) from Example 3 was added and the suspension boiled gently for 4 h before cooling to room temperature. The suspension obtained was poured into water, then extracted into ethyl acetate (3 x 200 ml) and the combined extracts washed with saturated sodium chloride solution, dried ($Na_2SO_4$) and concentrated to afford a pale tan solid. The solid was boiled in a small volume of ether (~20 ml), cooled and filtered to afford the benzothiazine as a cream solid (3.75 g, 79%). $\delta^1H$($d_6$ -DMSO) 7.21 (brs, 2H); 7.02 (d, J=2.0 Hz, 1H); 6.85 (brs, 1H); 3.52 (m, 2H); 2.89 (m, 2H).

Example 5

6-Chloro-1-oxo-8-sulfonamido-3,4-dihydro-2H-1,4-benzothiazine

3-Chloroperoxybenzoic acid (90%, 760 mg, 3.96 mmole) was added to a stirred ice-bath cooled solution of 6-chloro-8-sulfonamido-3,4-dihydro-2H-1,4-benzothiazine (1.0 g, 3.78 mmole). The reaction was stirred at ice-bath temperature for 10 min after which time the solvent was removed at reduced pressure. The residue was boiled in a small volume of ethyl acetate, cooled, filtered and washed with ether to afford on drying the sulfoxide as a colourless solid (0.86 g, 81%). $\delta^1H$ ($d_6$ -DMSO) 7.63 (brs, 1H); 7.54 (s, 2H); 6.95 (s, 2H); 3.58 (m, 2H); 3.10 (m, 2H).

Example 6

6-Chloro-1,1-dioxo-8-sulfonamido-3,4-dihydro-2H-1,4-benzothiazine

3-Chloroperoxybenzoic acid (90%, 1.17 g), 6.10 mmole) was added to a stirred solution of 6-chloro-8-sulfonamido-3,4-dihydro-2H-1,4-benzothiazine (735 mg, 2.78 mmole) in acetone (50 ml) all at room temperature. The reaction solution was stirred at room temperature for 6 h after which time the reaction volume was reduced (approx. 10 ml). Ether (50 ml) was added and the suspension boiled gently, following cooling to room temperature the suspension was filtered, washed with ether and dried to afford the sulfone as a colourless solid (0.69 g, 84%). $\delta^1H$ ($d_6$ -DMSO) 7.73 (brs, 1H); 7.16 (s, 2H); 7.11 (s, 2H); 3.70 (m, 2H); 3.52 (m, 2H).

Example 7

N-[(4-Methoxy-6-methylpyrimidin-2-yl)aminocarbonyl]-6-chloro-3,4-dihydro-2H-1,4-benzothiazine-8-sulfonamide (29)

A solution of 6-chloro-8-sulfonamido-3,4-dihydro-2H-1,4-benzothiazine (0.30 g, 1.13 mmole) and O-phenyl-N-(4-methoxy-6-methyl pyrimidin-2-yl) carbamate (353 mg, 1.36 mmole) in acetonitrile (20 ml) was treated with 1,8-diazabicyclo[5.4.0]undec-7-ene (0.25 ml). The mixture was stirred at room temperature for 5 days, the volume of the solution was reduced (approx. 10 ml) and water (50 ml) was added. The solution was then acidified with aqueous citric acid to pH 4. Following stirring at room temperature for a further 1 h, the suspension was filtered, washed with ether and dried to afford the sulfonyl urea (222 mg, 46%) as a colourless solid. $\delta^1H$ ($d_6$ -DMSO) 13.49 (S, 1H); 10.57 (S, 1H); 7.12 (s, 1H); 6.90 (s, 1H); 6.81 (s, 1H); 6.55 (s, 1H); 3.94 (s, 3H); 3.48 (m, 2H); 2.83 (m, 2H); 2.38 (s, 3H).

Example 8

N-[(4-methoxy-6-methylpyrimidin-2-yl)amino carbonyl]-6-chloro-1,1-dioxo-3,4-dihydro-2H-1,4-benzothiazine-8-sulfonamide (73)

A solution of O-phenyl-N-(4-methoxy-6-methylpyrimidin-2-yl)carbamate (524 mg, 2.02 mmole) and 6-chloro-1,1-dioxo-8-sulfonamido-3,4-dihydro-2H-1,4-benzothiazine (0.50 g, 1.68 mmole) in acetonitrile was treated with 1,8-diazobicyclo[5.4.0]undec-7-ene (0.30 ml) at room temperature for 39 h. Following work up as previously described the sulfonyl urea was obtained as a colourless solid (0.41 g, 53%). $\delta^1H(d_6$ -DMSO) 13.32 (s, 1H); 10.57 (s, 1H); 7.75 (s, 1H); 7.30 (d, J=2.2 Hz, 1H); 7.18 (d, J=2.2 Hz, 1H); 6.51 (s, 1H); 3.95 (s, 3H); 3.66 (m, 2H); 3.49 (m, 2H); 2.37 (s, 3H).

Example 9

N-[(4-Methoxy-6-methylpyrimidin-2-yl)amino carbonyl]-6-chloro-1-oxo-3,4-dihydro-2H-1,4-benzothiazine-8-sulfonamide (58)

A solution of O-phenyl-N-(4-methoxy-6-methylpyrimidin-2-yl)carbamate (0.44g, 1.71 mmole) and 6-chloro-1-oxo-8-sulfonamido-3,4-dihydro-2H-1,4-benzothiazine (0.40 g, 1.42 mmole) in acetonitrile was treated with 1,8-diazobicyclo[5.4.0]undec-7-ene (0.26 ml) at room temperature for 67 h. Following work up as outlined above the sulfonyl urea was obtained as a colourless solid (293 mg, 46%). $\delta^1H$ ($d_6$ -DMSO) 14.30 (brs, 1H); 10.68 (s, 1H); 7.71 (brs, 1H); 7.08 (s, 2H); 6.56 (s, 1H); 3.94 (s, 3H); 3.75 - 3.909 (m 4H); 2.39 (s, 3H).

Example 10

8-Sulfonamido-3,4-dihydro-2H-1,4-benzothiazin-3-one

A mixture of 6-chloro-8-sulfonamido-3,4-dihydro-2H-1,4-benzothiazin-3-one (from Example 3) (4.74 g, 17 mmole), 10% palladium on charcoal (1.00 g), formic acid (5 ml) and N,N-dimethylformamide was refluxed gently for 3h in a nitrogen atmosphere. Filtration of the mixture through Celite (diatomaceous earth), followed by rinsing of the Celite with ethyl acetate gave a solution which was evaporated in vacuo. The colourless crystalline residue was boiled in ethyl acetate (20 ml), cooled and filtered to afford the dechlorinated benzothiazinone as a colourless solid (4.11 g, 99%) $\delta^1H$ ($d_6$-DMSO) 10.34 (brs, 1H); 7.6-7.1 (m, 3H); 3.41 (s, 2H).

Example 11

8-Sulfonamido-3,4-dihydro-2H-1,4-benzothiazine

The sulfonamidobenzothiazinone (2.00 g, 8.2 mmole) from Example 10 was reacted with sodium borohydride (1.55 g)/aluminium chloride (1.82 g) in dimethoxyethane (50 ml) by the procedure described in Example 4 to give the title benzothiazine as a pale yellow solid (1.41 g, 75%). $\delta^1H$ ($d_6$-DMSO) 7.2-6.8 (m, 5H); 3.52 (m, 2H); 2.89 (m, 2H).

Example 12

Phenoxycarbonyl-3,4-dihydro-2H-1,4-benzothiazin-8-sulfonamide

8-Sulfonamido-3,4-dihydro-2H-1,4-benzothiazine (1.00 g, 4.3 mmole) was added to an ice-cooled mixture of sodium hydride (150 mg) and dry N,N-dimethylformamide (20 ml). The reaction mixture was stirred at 0°C for 30 min, allowed to warm to ambient temperature over 30 min, then diluted with water (30

ml) and acidified to pH 4 with aqueous citric acid. After extraction with ethyl acetate (2 x 50 ml), the organic phase was dried ($Na_2SO_4$) and concentrated at reduced pressure, then the excess phenol removed by distillation in a kugelrohr (70°C) and the residue titrated with ethyl acetate/ether to afford the title phenyl sulfonyl carbamate as a colourless powder (625 mg, 43%), which was used without further purification.

Example 13

N-[(4-Methoxy-6-methylpyrimidin-2-yl)aminocarbonyl)]-3,4-dihydro-2H-1,4-benzothiazine-8-sulfonamide (30)

8-Sulfonamido-3,4-dihydro-2H-1,4-benzothiazine (0.50 g, 2.2 mmole) was reacted with O-phenyl-N-(4-methoxy-6-methylpyrimidin-2-yl)carbamate (0.67 g; 2.6 mmole) in acetonitrile (30 ml) in the presence of 1,8-diazobicyclo[5.4.0]undec-7-ene (0.39 ml) according to the procedure described in Example 7 to give the title sulfonyl urea as a colourless solid (364 mg, 42%). $\delta^1H$ ($d_6$-DMSO) 13.26 (brs, 1H); 10.45 (s, 1H); 7.5-6.6 (m, 4H); 6.51 (s, 1H); 3.95 (s, 3H); 3.40 (m, 2H); 2.78 (m, 2H); 2.39 (s, 3H).

Example 14

N-[(4-Methoxy-6-methylpyrimidin-2-yl)aminocarbonyl]-1-oxo-3,4-dihydro-2H-1,4-benzothiazine-8-sulfonamide (46)

Reaction of 8-sulfonamido-1-oxo-3,4-dihydro-2H-1,4-benzothiazine (0.40 g, 1.6 mmole) with O-phenyl-N-(4-methoxy-6-methylpyrimidin-2-yl)carbamate (0.51 g, 2.0 mmole) according to the procedure described in Example 7 gave the title sulfoxide sulfonyl urea as an off-white powder (289 mg, 43%). $\delta^1H$ ($d_6$-DMSO) 13.75 (brs, 1H); 10.45 (s, 1H); 7.4-6.9 (m, 3H); 6.52 (s, 1H); 3.94 (s, 3H); 3.64 (m, 2H); 3.04 (m, 2H); 2.38 (s, 3H).

Example 15

N-[(4-Methoxy-6-methylpyrimidin-2-yl)aminocarbonyl]-1,1-dioxo-3,4-dihydro-2H-1,4-benzothiazine-8-sulfonamide (61)

Reaction of 8-sulfonamido-1,1-dioxo-3,4-dihydro-2H-1,4-benzothiazine (which was prepared in 86% yield from the parent sulfide by oxidation with 3-chloroperoxybenzoic acid by the method described in Example 6) (0.40 g, 1.5 mmole) with O-phenyl-N-(4-methoxy-6-methylpyrimidin-2-yl)carbamate (0.47 g, 1.8 mmole) according to the procedure described in Example 7 gave the title sulfone sulfonyl urea as a colourless solid (343 mg, 53%). $\delta^1H$ ($d_6$-DMSO) 13.05 (brs, 1H); 10.46 (s, 1H); 7.5-7.0 (m, 3H); 6.49 (s, 1H); 3.95 (s, 3H); 3.69 (m, 2H); 3.36 (m, 2H); 2.35 (s, 3H).

Example 16

N-[(4,6-Dimethoxypyrimidin-2-ylaminocarbonyl]-3,4-dihydro-2H-1,4-benzothiazine-8-sulfonamide (31)

A solution of phenoxycarbonyl-3,4-dihydro-2H-1,4-benzothiazin-8-sulfonamide (300 mg, 0.89 mmol) and 2-amino-4,6-dimethoxypyrimidine (150 mg, 0.96 mmol) in dry toluene (40 ml) was refluxed for 4 h. After distillation of the toluene, water (20 ml) was added and the solution acidified with aqueous citric acid to pH 4. Acetone (ca 20 ml) was added to make the mixture homogeneous, after which it was concentrated to ca 15-20 ml at reduced pressure. The precipitated solid was filtered, washed with water and dried to afford the title sulfonyl urea as a colourless powder (0.28 g, 77%). $\delta^1H$ ($CDCl_3$/$d_6$-DMSO) 12.78 (brs, 1H); 10.70 (brs, 1H); 9.30 (s, 1H); 7.6-6.9 (m, 3H); 5.77 (s, 1H); 3.98 (s, 6H); 3.63 (m, 2H); 2.93 (m, 2H).

Example 17

N-[(4,6-Dimethoxypyrimidin-2-yl)aminocarbonyl)]-1-oxo-3,4-dihydro-2H-1,benzothiazin-8-sulhonamide (47)

A solution of the sulfonyl urea sulphide (31) (from Example 16) (104 mg, 0.25 mmol) in acetone (20 ml) was treated with 3-chloroperoxybenzoic acid (90%, 48 mg, 0.28 mmol) at ice-bath temperature. The reaction was stirred at 0°C for 5 min, then concentrated in vacuo, and the residue titrated with ether. Filtration and drying afforded the sulfoxide sulfonyl urea as a colourless solid (0.80 g, 74%). $\delta^1H$ ($d_6$-DMSO) 12.98 (brs, 1H); 10.49 (s, 1H); 7.6-6.8 (m, 3H); 5.96 (s, 1H); 3.92 (s, 6H); 3.52 (m, 2H); 3.01 (m, 2H).

Example 18

N-[(4,6-Dimethoxypyrimidin-2-yl)aminocarbonyl]-1,1-dioxo-3,4-dihydro-2H-1,4-benzothiazin-8-sulfonamide (62)

3-Chloroperoxybenzoic acid (90%, 205 mg, 1.07 mmol) was added to a stirred solution of the sulfonyl urea sulfide (31) (from Example 16) (0.20 g, 0.49 mmol) in acetone (10 ml) all at room temperature. After stirring an additional 4 h, the solvent was evaporated at reduced pressure and the residue boiled in ether (approx 30 ml). Cooling, filtration an drying afforded the sulfone sulfonyl urea as a tan solid (0.18 g, 84%). $\delta^1H$ ($d_6$-DMSO) 12.75 (brs, 1H); 10.65 (brs, 1H); 7.4-6.9 (m, 3H); 5.90 (s, 1H); 3.95 (s, 6H); 3.67 (m, 2H); 3.45 (m, 2H).

Example 19

8-Amino-6-chloro-2-methyl-3,4-dihydro-2H-1,4-benzothiazin-3-one

1,4-Dichloro-2,6-dinitrobenzene (30 g, 0.125 mole) was reacted with thiolactic acid (12.3 ml) according to the procedure described in Example 1 for reaction with thioglycollic acid. The resultant 2-thiophenoxy propanoic acid (37.8 g, 98%) was then reacted with tin metal/hydrochloric acid in ethanol by the method described in Example 2. The resultant product (21.9 g) was dissolved in ethanol:5M sodium hydroxide (1:1, 940 ml) at 60°C under nitrogen and treated with sodium dithionite (37.5 g). After an additional 4 h warming, the mixture was allowed to slowly cool and then diluted with water and extracted several times with ethyl acetate. Drying of the organic extracts and evaporation afforded the methyl benzothiazinone as a fawn powder (13.2 g, 73%).

Example 20

6-Chloro-2-methyl-8-sulfonamido-3,4-dihydro-2H-1,4-benzothiazin-3-one and 6-chloro-2-methyl-8-t-butylsulfonamido-3,4-dihydro-2H-1,4-benzothiazine-3-one

8-Amino-6-chloro-2-methyl-3,4-dihydro-2H-1,4-benzothiazin-3-one was reacted by the method described in Example 3 to give 6-chloro-2-methyl-8-sulfonamido-3,4-dihydro-2H-1,4-benzothiazin-3-one in 50% yield.

By using a similar procedure, but replacing the aqueous ammonia/ether by three equivalents of t-butylamine in acetonitrile (at 0°C) the t-butylsulfonamide was produced in 49% yield.

Example 21

2-Methyl-8-sulfonamide-3,4-dihydro-2H-1,4-benzothiazine

Sequential dechlorination (using 10% palladium on charcoal in a 1:10 mixture of formic acid and N,N-dimethylformamide in a Parr hydrogenator) of the 8-sulfonamido compound of Example 19; followed by reduction of the amido carbonyl as described in Example 4, afforded the title benzothiazine as a tan solid.

Example 22

2,4-Dimethyl-8-sulfonamido-3,4-dihydro-2H-1,4-benzothiazine

6-Chloro-2-methyl-8-t-butylsulfonamido-3,4-dihydro-2H-1,4-benzothiazin-3-one was sequentially N-methylated (by room temperature rection with methyl iodide and potassium carbonate in acetone, 93%); dechlorinated (using 10% palladium on charcoal in a 1:10 formic acid:DMF mixture in a Parr hydrogenator, quantitative); de-t-butylated (by heating in trifluoroacetic acid at 50° for 4 h, 53%); and reduced (with aluminium chloride/sodium borohydride in dimethoxyethane, quantitative) to afford the title dimethyl benzothiazine as white crystals. $\delta^1H$ NMR ($d_6$ - acetone) 7.35-6.5 (m, 8H); 6.24 (brs, 2H); 3.62 (m, 1H); 3.29 (m, 2H); 3.00 (s, 3H); 1.36 (d, j = 6.6Hz, 3H).

Example 23

Compounds Nos. 33 and 34 were each prepared by reaction of the O-phenyl carbamate of the appropriate 2-aminopyrimidine with 2-methyl-8-sulfonamido-3,4-dihydro-2H-1,4-benzothiazine (from Example 21). Compound No. 49 was obtained by oxidation of Compound No. 33 with 3-chloroperoxybenzoic acid.

Compound 33:

$\delta^1H$ ($d_6$-DMSO) 13.33 (s, 1H); 10.50 (s, 1H); 7.23 (dd, J=6.8, 1.2 Hz, 1H); 7.02 (t, J=7.9 Hz, 1H); 6.79 (dd, J=7.0, 1.2 Hz, 1H); 6.68 (brs, 1H); 6.56 (s, 1H); 3.95 (s, 3H); 3.55 (m, 1H); 3.07 (m, 2H); 2.39 (s, 3H); 1.03 (d, J=6.1 Hz, 3H).

Compound 34:

$\delta^1H$ ($d_6$-DMSO) 12.85 (s, 1H); 10.62 (s, 1H); 9.32 (s, 1H); 7.16 (m, 1H); 6.8-6.7 (m, 2H); 6.03 (s, 1H); 3.95 (s, 6H); 3.59 (m, 1H); 3.13 (m, 2H); 1.09 (d, J = 6.0 Hz, 3H).

Compound 49:

$\delta^1H$ ($d_6$-DMSO) 13.76 (brs, 1H); 10.50 (s, 1H); 7.6-7.0 (m, 3H); 6.55 (s, 1H); 3.94 (s, 3H); 3.40 (m, 3H); 2.39 (s, 3H); [1.06 (d, J=7.0 Hz) and 0.64 (d, J=7.0 Hz) 1H].

Example 24

Compound Nos. 39 and 55 were each prepared from 2,4-dimethyl-8-sulfonamido-3,4-dihydro-2H-1,4-benzothiazine (from Example 22). Direct coupling of the sulfonamide with the O-phenyl carbamate of 2-amino-4-methoxy-6-methylpyrimidine; subsequent oxidation with 3-chloroperoxybenzoic acid generated compound No. 55.

Compound 39:

$\delta^1H$ ($d_6$-DMSO) 13.39 (brs, 1H); 10.49 (s, 1H); 7.38 (d, J=7.7 Hz, 1H); 7.19 (t, J=8.1 Hz, 1H); 6.98 (d, J=8.3 Hz, 1H); 6.55 (s, 1H); 3.95 (s, 3H); 3.60 (m, 1H); 3.17 (m, 2H); 2.95 (s, 3H), 2.39 (s, 3H); 1.04 (d, J=5.9 Hz, 3H).

Compound 55:

$\delta^1H$ ($d_6$-DMSO) 13.64 (brs, 1H); 10.54 (s, 1H); 7.7-7.05 (m, 3H); 6.56 (s, 1H); 3.95 (s, 3H); 3.12 (s, 3H); 2.38 (s, 3H); [1.13 (d, J=6.7 Hz) and 0.64 (d, J=6.7 Hz) 1H].

Example 25

N-[(4-Methoxy-6-methylpyrimidin-2-yl)aminocarbonyl]-6-chloro-2-methyl-3,4-dihydro-2H-1,4-benzothiazine-8-sulfonamide (42)

6-Chloro-2-methyl-8-sulfonamido-3,4-dihydro-2H-1,4-benzothiazin-3-one (from Example 20) (1.00 g, 3.4 mmole) was reduced with aluminium chloride/sodium borohydride in dimethoxyethane by the method described in Example 4, to give the benzothiazine sulfonamide as an off-white powder (0.92 g, 97); which was coupled with O-phenyl-N-(4-methoxy-6-methylpyrimidin-2-yl)carbamate according to the procedure described in Example 7 to give the title sulfonyl urea as an off-white solid in 10% yield. $\delta^1$H NMR ($d_6$-DMSO) 10.96 (s, 1H); 9.45 (s, 1H); 8.42 (s, 1H); 7.5-6.5 (m, 3H); 6.55 (s, 1H); 3.97 (s, 3H); 3.50 (m, 3H); 2.41 (s, 3H); 1.14 (d, J-6.0 Hz, 3H).

Example 26

N-t-butyl 5-bromo-2-hydroxy-3-nitrobenzene-sulfonamide

(i) 4-Bromophenol (50.0 g) was added portionwise to ice-cooled, vigorously stirred chlorosulfonic acid (135 ml) at such a rate that the temperature remained below ca 5°C. After stirring at ambient temperature ovenight the mixture was cautiously poured over ice to give the crude 5-bromo-2-hydroxybenzenesulfonyl chloride.

(ii) A solution of this crude product in dichloromethane (200 ml) was added dropwise with cooling (5-20°C) to a stirred mixture of concentrated sulfuric acid and concentrated nitric acid (1:1, 44 ml). The mixture was left stirring for 2 h then poured into ice-water and extraced with dichloromethane to give crude 5-bromo-2-hydroxy-3-nitrobenzenesulfonyl chloride as a yellow oil.

(iii) t-butylamine (70 ml) was added dropwise to an ice-cooled solution of the crude nitrobenzenesulfonyl chloride in dichloromethane (ca 250 ml). The mixture was stirred at ambient temperature overnight and then evaporated at reduced pressure. The residue was shaken with ethyl acetate (500 ml) and aqueous citric acid (4%, 500 ml). The organic phase was separated, dried ($Na_2SO_4$) and evaporated to give a solid residue which was recrystallized from aqueous ethanol to give the title compound as a yellow solid (37.0 g). $\delta^1$H NMR ($CDCl_3$) 8.41 (d, J=2 Hz, 1H); 8.36 (d, J=1 Haz, 1H); 5.11 (brs, 1H); 1 .27 (s, 9H).

Example 27

8-t-Butylsulfonamido-3,4-dihydro-2H-1,4-benzoxazin-3-one

(i) Ammonium formate (30.0 g) was added to a stirred and cooled mixture of N-6-butyl 5-bromo-2-hydroxy-3-nitrobenzenesulfonamide (22.0 g) and 10% palladium on charcoal (2.0 g) in dry methanol (200 ml) under nitrogen. The mixture was stirred at ambient temperature overnight and then filtered through diatomaceous earth. The filtrate was concentrated and quickly taken up into a mixture of ethyl acetate and water. The organic layer was separated, washed with brine and evaporated to leave N-t-butyl 3-amino-2-hydroxybenzenesulfonamide as a labile amber oil. $\delta^1$H NMR ($d_6$-acetone) 7.1-6.6 (m, 3H); 1:1 (s, 9H).

(ii) The crude aminophenol was reacted, without delay, with chloroacetyl chloride according to the general procedure described in Organic Preparations and Procedures Int. 14 (3), 195-224 (1982) to give the title compound as a white solid (10.4 g). $\delta^1$H NMR ($d_6$-DMSO) 11.0 (brs, 1H); 7.5-6.9 (m, 4H); 4.7 (s, 2H); 1.1 (s, 9H).

Example 28

N-[(4-Methoxy-6-methylpyrimidin-2-yl)aminocarbonyl]-3,4-dihydro-2H-1,4-benzoxazine-8-sulfonamide (2)

(i) Lithium aluminium hydride (2.5 g) was added to a stirred suspension of 8-t-butylsulfonamido-3,4-dihydro-2H-1,4-benzoxazin-3-one (4.0 g) in diethyl ether (300 ml) under an atmosphere of dry nitrogen. The reaction mixture was heated under reflux for 5 hours and then cooled and quenched carefully with water. The mixture was neutralized with dilute hydrochloric acid and the ether layer was separated and dried ($MgSO_4$) to give 8-t-butylsulfonamido-3,4-dihydro-2H-1,4-benzoxazine (3.3g). $\delta^1$H NMR ($d_6$-acetone) 6.95 (m, 1H); 6.74 (m, 2H) 4.28 (t, J=4 Hz, 2H); 3.40 (m, 2H); 1.16 (s, 9H). The t-butyl group was removed by treating the N-t- butylsulfonamide (3.0 g) with trifluoroacetic acid (20 ml) at room temperature for several days.

(ii) Reaction of 8-sulfonamido-3,4-dihydro-2H-1,4-benzoxazine from part (i) (0.50 g) with O-phenyl-N-(4-methoxy-6-methylpyrimidin-2-yl) carbamate (0.83 g) following essentially the same procedure as given in Example 7 gave the sulfonylurea compound No. 2 as a white solid. (0.53 g). δ$^1$H($d_6$-acetone) 13.0 (s, 1H); 11.2(s, 1H), 9.1 (s, 1H); 7.3-6.7(m, 3H); 4.1(m,2H); 3.9(s, 3H); 3.4(m, 2H); 2.4(s, 3H).

Example 29

N-[(4-Methoxy-6-methylpyrimidin-2-yl)aminocarbonyl)-4-formyl-3,4-dihydro-2H-1,4-benzoxazine-8-sulfonamide (82)

(1) 8-t-Butylsulfonamido-3,4-dihydro-2H-1,4-benzoxazine (from Example 28) (1.50 g) was refluxed in 98% formic acid for 2 h. The mixture was then diluted with water and extracted with ethyl acetate. Successive washing of the organic phase with water and saturated brine, followed by filtration and evaporation of the solvent gave a residual soil, which was crystallized from ethyl acetate/diethyl ether to give 4-formyl-8-sulfonamido-3,4-dihydro-2H-1,4-benzoxazine as a white powder (0.29 g). δ$^1$H ($d_6$-acetone) 8.87 (S, 1H); 8.6-6.8 (m, 3H); 6.40 (brs, 2H); 4.42 m, 2H); 3.94 (m, 2H).

(ii) Reaction of the 4-formyl sulfonamide from part (i) (0.26 g) with O-phenyl-N-(4-methoxy-6-methylpyrimidin-2-yl) carbamate (0.59 g) following essentially the same procedure as given in Example 7 gave the sulfonyl urea·compound No. 82 as a white solid (0.29 g). δ$^1$H ($d_6$-acetone/$d_6$-DMSO) 13.3 (brs, 1H); 9.9 (brs, 1H); 8.8-7.0 (m, 3H); 6.5 (s, 1H); 4.3 (m, 2H); 3.9 (m, 5H); 2.4 (s, 3H).

Example 30

N-[(4-Methoxy-6-methylpyrimidin-2-yl)aminocarbonyl]-4-acetyl-3,4-dihydro-2H-1,4-benzoxazine-8-sulfonamide (83)

(i) 8-t-butylsulfonamido-3,4-dihydro-2H-1,4-benzoxazine (from Example 28) (2.00 g) was stirred at ambient temperature with acetic anhydride (3.5 ml), pyridine (20 ml) and a catalytic quantity of N,N-dimethylaminopyridine overnight. The reaction mixture was partitioned between ethyl acetate and aqueous citric acid and the organic phase separated. Washing with citric acid (thrice), saturated brine and evaporation gave an oil which was crystallized with diethyl ether to give 4-acetyl-8-t-butyl-sulfonamido-3,4-dihydro-2H-1,4-benzoxazine as a white solid (2.07 g). δ$^1$H ($d_6$-acetone) 7.82 (m, 1H); 7.54 (dd, J=7.6, 1.5 Hz, 1H); 6.94 (t, J=7.6 Hz, 1H); 6.17 (b, 1H); 4.48 (t, J=5.1 Hz, 2H); 3.95 (t, J=5.1 Hz, 2H); 2.28 (s, 3H); 1.16 (s, 9H).

(ii) The t-butylsulfonamide from part (i) (2.00 g) was warmed at 60°C in trifluoroacetic acid for 2 h; the mixture was then evaporated and the residue triturated with ethyl acetate and filtered to give 4-acetyl-8-sulfonamido-3,4-dihydro-2H-1,4-benzoxazine as a white solid (1.00 g). δ$^1$H ($d_6$-acetone/$d_6$-DMSO) 7.87 (m, 1H); 7.51 (dd, J=9, 1 Hz, 1H); 6.94 (m, 3H); 4.45 (t, J=5 Hz, 2H); 3.93 (t, J=5 Hz, 2H); 2.27 (s, 3H).

(iii) Reaction of the 4-acetyl sulfonamide from part (ii) (0.50 g) with O-phenyl-N-(4-methoxy-6-methylpyrimidin-2-yl) carbamate (0.70 g) following essentially the same procedure as in Example 7 gave the sulfonyl urea compound NO. 83 as a white solid (0.47 g). δ$^1$H ($d_6$-acetone/$d_6$-DMSO) 13.2 (brs, 1H); 10.3 (brs, 1H); 8.3-6.9 (m, 3H); 6.5 (s, 1H); 4.3 (m, 2H); 3.9 (m, 5H); 2.4 (s, 3H); 2.2 (s, 3H).

Example 31

N-[(4-methoxy-6-methylpyrimidin-2-yl)aminocarbonyl)]-4-methyl-3,4-dihydro-2H-1,4-benzoxazine-8-sulfonamide (19)

(i) 8-t-Butylsulfonamido-3,4-dihydro-2H-1,4-benzoxazin-3-one (from Example 27) (2.54 g) was refluxed with methyl iodide (2.8 ml) and potassium carbonate (1.,80 g) in acetone overnight. After evaporation of the acetone, the residue was partitioned between ethyl acetate and aqueous citric acid. Separation of the organic phase, followed by washing (with water and saturated brine) and evaporation gave 8-t-butylsulfonamido-4-methyl-3,4-dihydro-2H-1,4-benzoxazin-3-one. δ$^1$H ($CDCl_3$) 7.7 (m, 1H); 7.0 (m, 2H); 5.0 (s, 1H); 4.7 (s, 2H); 3.4 (s, 3H); 1.3 (s, 9H).

(ii) Lithium aluminium hydride (1.30 g) was added to the stirred crude amide from part (i) in dioxane

under an atmosphere of dry nitrogen. The reaction mixture was warmed at 100°C for 6 h and then cooled and quenched carefully with a mixture of ethyl acetate and aqueous citric acid. The mixture ws neutralized (to pH 7) with dilute hydrochloric acid and the organic phase separated and evaporated. Trituration of the residue with diethyl ether/hexane then afforded 8-t-butylsulfonamido-4-methyl-3,4-dihydro-2H-1,4-benzoxazine as a white solid. $\delta^1H$ ($d_6$-acetone) 7.05 (t, J=5 Hz, 1H); 6.82 (m, 2H); 5.87 (brs, 1H); 4.39 (t, J=5 Hz, 2H); 3.34 (t, J=5 Hz, 2H); 2.92 (s, 3H); 1.16 (s, 9H).

The t-butyl group was removed by treating the crude N-t-butylsulfonamide with trifluoroacetic acid at 60-70°C for 1.5 h to give 8-sulfonamido-4-methyl-3,4-dihydro-2H-1,4-benzoxazine as an off-white solid (0.80 g).

(iii) Reaction of the 4-methyl sulfonamide from part (ii) (0.80 g) with O-phenyl-N-(4-methoxy-6-methylpyrimidin-2-yl) carbamate (2.06 g) following essentially the same procedure as given in Example 7 gave the sulfonylurea compound No. 19 as a white solid (0.87 g). $\delta^1H$ ($d_6$-acetone/$d_6$-DMSO) 13.0 (brs, 1H); 9.8 (brs, 1H); 7.4 (m, 1H); 6.7 (m, 2H); 6.5 (s, 1H); 4.2 (m, 2H); 3.9 (s, 3H); 3.3 (m, 2H); 2.9 (s, 3H); 2.4 (s, 3H).

## Example 32

This non-limiting Example illustrates the preparation of formulations of the compounds of the invention.

a) Emulsifiable Concentrate

Compound No. 2 was dissolved in toluene/DMSO containing 7% v/v "Teric" N13 and 3% v/v "Kemmat" SC15B to give an emulsifiable concentrate which was diluted with water to the required concentration to give an aqueous emulsion which was applied by spraying.

("Teric" is a Trade Mark and "Teric" N13, is a product of ethoxylation of nonylphenol; "Kemmat" is a Trade Mark and "Kemmat" SC15B is a formulation of calcium dodecylbenzenesulfonate).

b) Aqueous Suspension

Compound No 1 (5 parts by weight) and "Dyapol" PT (1 part by weight) were added to a 2% aqueous solution (94 parts by weight) of "Teric" N8 and the mixture was ball milled to produce a stable aqueous suspension which may be diluted with water to the required concentration to give an aqueous suspension which may be applied by spraying.

("Dyapol" is a Trade Mark and "Dyapol" PT is an anionic suspending agent; "Teric" N8 is a product of ethoxylation of nonylphenol.)

c) Emulsifiable Concentrate

Compound No 30 (10 parts by weight), "Teric" N13 (5 parts by weight) and "Kemmat" SC15B (5 parts by weight) were dissolved in "Solvesso" 150 (50 parts by weight) to give an emulsifiable concentrate which may be diluted with water to the required concentration to give an aqueous emulsion which may be applied by spraying.

("Solvesso" is a Trade Mark and "Solvesso" 150 is a high boiling point aromatic petroleum fraction.)

d) Dispersible Powder

Compound No. 1 (10 parts by weight), "Matexil" DA/AC (3 parts by weight), "Aerosol" OT/B (1 part by weight) and china clay 298 (86 parts by weight) were blended and then milled to give a powder composition having a particle size below 50 microns. ("Matexil" is a Trade Mark and "Matexil" DA/AC is the disodium salt of a naphthalenesulfonic acid/formaldehyde condensate; "Aerosol" is a Trade Mark and "Aerosol" OT/B is a formulation of the dioctyl ester of sodium sulfosuccinic acid.)

e) Dusting Powder

Compound No 1 (10 parts by weight), attapulgite (10 parts by weight) and pyrophyllite (80 parts by weight) were thoroughly blended and then ground in a hammer-mill to produce a powder of particle size less than 200 microns.

Emulsifiable concentrates and/or suspensions of the compounds of the invention were prepared essentially as described in part a), b) or c) above and then diluted with water, optionally containing surface active agent and/or oil, to give aqueous compositions of the required concentration which were used, as described in Example 33, in the evaluation of the post-emergence herbicidal activity of the compounds.

## Example 33

The post-emergent herbicidal activity of the compounds of the invention formulated as described in Example 32 was assessed by the following procedure.

The seeds of the test species were sown in rows 2 cm deep in soil contained in seed boxes. The monocotyledonous plants and the dicotyledonous plants were sown in separate seed boxes in duplicate.

The four seed boxes were placed in a glass house, lightly watered with an overhead spray to initiate germination and then sub-irrigated as required for optimum plant growth. After the plants had grown to a height of about 10 to 12.5 cm one box of each of the monocotyledonous plants and the dicotyledonous plants was removed from the glass house and sprayed with the required quantity of a composition of the invention. After spraying the boxes were returned to the glass house for a further 3 weeks and the effect of treatment was visually assessed by comparison with the untreated controls. The results are presented in Table 2 where the damage to plants is rated on a scale of from 0 to 5 where 0 represents from 0 to 10% damage, 2 represents from 31 to 60% damage, 3 represents from 61 to 80% damage, 4 represents from 81 to 99% damage and 5 represents 100% kill. A dash (-) means that no experiment was carried out.

The names of the test plants are as follows:

| | |
|---|---|
| Wh | Wheat |
| Ot | Wild Oats |
| Rg | Ryegrass |
| Jm | Japanese millet |
| B | Barley |
| P | Peas |
| Ip | Ipomea |
| Ms | Mustard |
| Sf | Sunflower |
| Mz | Maize |

TABLE 2

Post-emergence Herbicidal Activity

| Compound No. | Application Rate kg/ha | Wh | Jm | Rg | Ot | B | P | Ip | Ms | Sf | Mz |
|---|---|---|---|---|---|---|---|---|---|---|---|
| 2 | 0.4 | 4 | 3 | 3 | 4 | 4 | 3 | 4 | 4 | 5 | 4 |
| 2 | 0.1 | - | - | - | - | - | 2 | 3 | 4 | 5 | - |
| 19 | 0.4 | 3 | 2 | 1 | 3 | 4 | 1 | 2 | 4 | 3 | 3 |
| 19 | 0.1 | - | - | - | - | - | 0 | 3 | 2 | 2 | - |
| 29 | 0.4 | 2 | 2 | 0 | 3 | 0 | 3 | 5 | 5 | 5 | 3 |
| 30 | 0.4 | 5 | 4 | 5 | 5 | 4 | 3 | 5 | 5 | 5 | 4 |
| 30 | 0.1 | - | - | - | - | - | 4 | 5 | 5 | 4 | - |
| 30 | 0.025 | - | - | - | - | - | 3 | 4 | 4 | 4 | - |
| 33 | 0.4 | 4 | 1 | 2 | 3 | 3 | 3 | 4 | 5 | 4 | 2 |
| 33 | 0.1 | - | - | - | - | - | 2 | 3 | 3 | 4 | - |
| 34 | 0.4 | 3 | 4 | 1 | 4 | 3 | 2 | 4 | 2 | 4 | 5 |
| 39 | 0.4 | 4 | 2 | 4 | 4 | 4 | 3 | 4 | 4 | 3 | 3 |

TABLE 2 (Continued)

Post-emergence Herbicidal Activity

| Compound No. | Application Rate kg/ha | Wh | Jm | Rg | Ot | B | P | Ip | Ms | Sf | Mz |
|---|---|---|---|---|---|---|---|---|---|---|---|
| 39 | 0.1 | - | - | - | - | - | 3 | 3 | 3 | 3 | - |
| 46 | 0.1 | 4 | 0 | 0 | 3 | 2 | 0 | 1 | 3 | 3 | 4 |
| 49 | 0.4 | 4 | 3 | 4 | 4 | 4 | 3 | 4 | 4 | 3 | 4 |
| 49 | 0.1 | - | - | - | - | - | 1 | 2 | 2 | 3 | - |
| 58 | 0.4 | 4 | 2 | 1 | 4 | 4 | 3 | 5 | 5 | 4 | 3 |
| 61 | 0.1 | 4 | 1 | 3 | 4 | 3 | 0 | 2 | 4 | 4 | 3 |
| 61 | 0.025 | 4 | - | 1 | 3 | 3 | 0 | 2 | 5 | 3 | 0 |
| 62 | 0.4 | 4 | 4 | 4 | 4 | 4 | 4 | 4 | 4 | 5 | 5 |
| 62 | 0.1 | - | - | - | - | - | 3 | 3 | 4 | 5 | - |
| 73 | 0.4 | 1 | 3 | 0 | 1 | 1 | 3 | 5 | 5 | 5 | 4 |
| 73 | 0.1 | - | - | - | - | - | 1 | 3 | 2 | 3 | - |
| 82 | 0.4 | 3 | 2 | 2 | 3 | 4 | 3 | 4 | 4 | 5 | 4 |
| 82 | 0.1 | - | - | - | - | - | 1 | 3 | 4 | 4 | - |
| 82 | 0.025 | - | - | - | - | - | 0 | 2 | 2 | 3 | - |
| 83 | 0.4 | 4 | 3 | 3 | 4 | 4 | 2 | 3 | 4 | 5 | 4 |
| 83 | 0.1 | - | - | - | - | - | 1 | - | 4 | 4 | - |

Example 34

The compounds were formulated for test by mixing an appropriate amount with 5 ml of an emulsion prepared by diluting 160 ml of a solution containing 21.9 g per litre of "Span" 80 and 78.2 g per litre of "Tween" 20 in methylcyclohexanone to 500 ml with water. "Span" 80 is a Trade Mark for a surface-active agent comprising sorbitan monolaurate. "Tween" 20 is a Trade Mark for a surface-active agent comprising a condensate of sorbitan monolaurate with 20 molar proportions of ethylene oxide. Each 5 ml emulsion containing a test compound was then diluted to 40 ml with water and sprayed on to young pot plants (post-emergence test) of the species named in Tables 3 and 4 below. Damage to test plants was assessed after 13 days. In a test for pre-emergence herbicidal activity, seeds of the test plants were sown in a shallow slit formed in the surface of soil in fibre trays. The surface was then levellev and sprayed, and fresh soil then spread thinly over the sprayed surface. Assessment of herbicidal damage, recorded in Table 5 below, was carried out after 20 days. In Tables 3 and 5 assessment was of 0 to 5 wherein 0 is 0 to 20% damage and 5 is complete kill. In Table 4 assessment was on a 0 to 9 scale where

0 represents zero damage,
1 represents from 1 to 5% damage,
3 represents from 16 to 25% damage,
4 represents from 26 to 35% damage,

5 represents from 36 to 59% damage,
6 represents from 60 to 69% damage,
7 represents from 70 to 79% damage,
8 represents from 80 to 89%.damage and
9 represents from 90 to 100% damage.

The degree of herbicidal damage was assessed by comparison with untreated control plants. The results are given in Tables 3 to 6 below. A dash (-) means no experiment was carried out.

The names of the test plants were as follows:

| | |
|---|---|
| Sb | Sugar Beet |
| Rp | Oilseed Rape |
| Ct | Cotton |
| Sy | Soya Bean |
| Mz | Maize |
| Ww | Winter wheat |
| Rc | Rice |
| Bd | Bidens pilosa |
| Ip | Ipomea |
| Am | Amaranthus retroflexus |
| Pi | Polygonum |
| Ca | Chenopodium album |
| Ga | Galium aparine |
| Xs | Xanthium spinosum |
| Ab | Abutilon theophrasti |
| Co | Cassia obtusifolia |
| Av | Avena fatua |
| Dg | Digitaria sanquinalis |
| Al | Alopecurus myosuroides |
| St | Setaria viridis |
| Sh | Sorghum halepense |
| Ag | Agropyron repens |
| Cn | Cyperus rotundus |
| Xa | Xanthium pensylvanicum |
| Ce | Cyperus esculentis |
| Eh | Euphorbia heterophylla |

TABLE 3

Post-Emergent Herbicidal Activity

| Compound No. | 2 | 2 | 2 | 2 | 19 | 19 | 29 | 29 | 30 | 30 | 30 |
|---|---|---|---|---|---|---|---|---|---|---|---|
| Rate g/ha | 625 | 125 | 25 | 5 | 625 | 125 | 125 | 25 | 125 | 25 | 5 |
| Test Plants | | | | | | | | | | | |
| Sb | 4 | 4 | 4 | 2 | 3 | 2 | 2 | 1 | 3 | 3 | 2 |
| Rp | 5 | 5 | 4 | 4 | – | 4 | 4 | 3 | 4 | 4 | 3 |
| Ct | 3 | 2 | 1 | 0 | 2 | 0 | 3 | 2 | 2 | 1 | 0 |
| Sy | 5 | 5 | 2 | 1 | 3 | 2 | 3 | 1 | 4 | 4 | 4 |
| Mz | 5 | 4 | 4 | 3 | 4 | 3 | 3 | 2 | 4 | 3 | 3 |
| Ww | 4 | 4 | 3 | 3 | 3 | 3 | 1 | 2 | 4 | 3 | 2 |
| Rc | 2 | 2 | 1 | 0 | 1 | 1 | 4 | 3 | 2 | 1 | 0 |
| Bd | 5 | 5 | 5 | 4 | 4 | 3 | 4 | 3 | 5 | 5 | 3 |
| Ip | 5 | 4 | 4 | 3 | 3 | 3 | 1 | 2 | 3 | 3 | 3 |
| Am | 5 | 5 | 4 | 4 | 5 | 4 | 4 | 3 | 5 | 5 | 4 |
| Pi | 4 | 4 | 3 | 2 | 3 | 3 | 2 | 1 | 3 | 1 | 1 |
| Ca | 5 | 5 | 4 | 2 | 3 | 3 | 1 | 0 | 3 | 3 | 2 |
| Ga | 5 | 5 | 4 | 4 | 3 | 3 | 3 | 3 | 5 | 4 | 2 |
| Xs | 5 | 5 | 5 | 1 | 4 | 3 | 3 | 2 | 5 | 4 | 3 |
| Ab | 5 | 5 | 3 | 1 | 3 | 3 | 3 | 1 | 4 | 3 | 2 |
| Co | 3 | 1 | 1 | 0 | 3 | 2 | 2 | 2 | 2 | 1 | 1 |
| Av | 5 | 5 | 4 | 3 | 4 | 2 | 2 | 2 | 4 | 3 | 2 |
| Dg | 5 | 5 | 4 | 1 | 4 | 3 | 1 | 1 | 4 | 2 | 0 |
| Al | 5 | 4 | 4 | 3 | 3 | 2 | 1 | 2 | 3 | 2 | 2 |
| St | 5 | 5 | 2 | 1 | 3 | 3 | 0 | 1 | 4 | 3 | 0 |
| Ec | 5 | 4 | 4 | 4 | 3 | 3 | 1 | 3 | 4 | 3 | 2 |
| Sh | 5 | 5 | 5 | 4 | 3 | 2 | 3 | 2 | 4 | 4 | 3 |
| Ag | 4 | 4 | 4 | 3 | 3 | 3 | 1 | 2 | 4 | 4 | 3 |
| Cn | 3 | 3 | 3 | 0 | 3 | 3 | 0 | 0 | 2 | 0 | 0 |

TABLE 3 (Continued)

Post-Emergent Herbicidal Activity

| Compound No. | 31 | 31 | 31 | 31 | 33 | 33 | 33 | 39 | 39 | 39 |
|---|---|---|---|---|---|---|---|---|---|---|
| Rate g/ha | 625 | 125 | 25 | 5 | 625 | 125 | 25 | 625 | 125 | 25 |
| Test Plants | | | | | | | | | | |
| Sb | 5 | 4 | 4 | 3 | 4 | 4 | 3 | 4 | 3 | 2 |
| Rp | 5 | 4 | 4 | 4 | 5 | 5 | 3 | 5 | 5 | 4 |
| Ct | 4 | 3 | 1 | 1 | 1 | 3 | 1 | 3 | 3 | 0 |
| Sy | 5 | 5 | 5 | 1 | 5 | 5 | 5 | 5 | 5 | 4 |
| Mz | 4 | 3 | 3 | 1 | 4 | 4 | 4 | 4 | 4 | 4 |
| Ww | 4 | 3 | 3 | 1 | 4 | 3 | 2 | 3 | 3 | 3 |
| Rc | 3 | 2 | 1 | 1 | 3 | 3 | 0 | 1 | 1 | 1 |
| Bd | 5 | 5 | 5 | 4 | 5 | 5 | 4 | 5 | 5 | 4 |
| Ip | 4 | 3 | 2 | 0 | 3 | 2 | 0 | 4 | 4 | 3 |
| Am | 5 | 5 | 5 | 4 | 5 | 5 | 4 | 5 | 4 | 4 |
| Pi | 4 | 4 | 4 | 4 | 4 | 3 | 2 | 3 | 2 | 2 |
| Ca | 5 | 5 | 4 | 0 | 5 | 4 | 4 | 4 | 4 | 3 |
| Ga | 5 | 5 | 4 | 3 | 5 | 3 | 2 | 5 | 5 | 3 |
| Xs | 5 | 5 | 5 | 1 | 5 | 4 | 1 | 5 | 4 | 3 |
| Ab | 5 | 4 | 4 | 1 | 5 | 4 | 1 | 3 | 3 | 1 |
| Co | 5 | 4 | 2 | 0 | 4 | 3 | 1 | 5 | 4 | 3 |
| Av | 5 | 5 | 4 | 3 | 4 | 4 | 4 | 4 | 4 | 2 |
| Dg | 5 | 4 | 1 | 0 | 4 | 3 | 1 | 4 | 4 | 3 |
| Al | 4 | 4 | 3 | 1 | 4 | 3 | 0 | 4 | 4 | 3 |
| St | 5 | 4 | 4 | 2 | 4 | 2 | 1 | 2 | 2 | 0 |
| Ec | 5 | 5 | 5 | 3 | 4 | 4 | 3 | 4 | 4 | 1 |
| Sh | 5 | 5 | 5 | 2 | 4 | 4 | 3 | 4 | 4 | 3 |
| Ag | 4 | 3 | 3 | 2 | 4 | 3 | 2 | 2 | 2 | 1 |
| Cn | 5 | 3 | 1 | 0 | 2 | 3 | 0 | 2 | 2 | 1 |

TABLE 3 (Continued)

Post-Emergent Herbicidal Activity

| Compound No. | 42 | 42 | 46 | 46 | 47 | 49 | 49 | 55 | 55 | 55 | 55 | 58 |
|---|---|---|---|---|---|---|---|---|---|---|---|---|
| Rate g/ha | 125 | 25 | 125 | 25 | 125 | 125 | 25 | 625 | 125 | 25 | 5 | 125 |
| Test Plants | | | | | | | | | | | | |
| Sb | 4 | 2 | 4 | 3 | 4 | 4 | 4 | 4 | 4 | 4 | 4 | 3 |
| Rp | 4 | 3 | 4 | 3 | 5 | 4 | 4 | 5 | 5 | 4 | 3 | 4 |
| Ct | 2 | 0 | 3 | 1 | 1 | 3 | 4 | 4 | 4 | 2 | 1 | 1 |
| Sy | 3 | 2 | 4 | 4 | 5 | 4 | 4 | 5 | 4 | 1 | 1 | 3 |
| Mz | 4 | 2 | 4 | 3 | 4 | 4 | 3 | 4 | 4 | 4 | 3 | 4 |
| Ww | 2 | 0 | 4 | 3 | 4 | 4 | 4 | 4 | 3 | 2 | 1 | 2 |
| Rc | 0 | 0 | 2 | 0 | 5 | 3 | 3 | 1 | 1 | 0 | 0 | 1 |
| Bd | 4 | 3 | 4 | 4 | 5 | 5 | 4 | 5 | 4 | 4 | 4 | 3 |
| Ip | 2 | 2 | 4 | 3 | 4 | 4 | 3 | 4 | 3 | 3 | 2 | 3 |
| Am | 5 | 3 | 4 | 4 | 5 | 5 | 4 | 5 | 5 | 4 | 4 | 3 |
| Pi | 1 | 1 | 1 | 1 | 4 | 4 | 4 | 4 | 4 | 2 | 2 | 1 |
| Ca | 3 | 2 | 4 | 2 | 5 | 4 | 5 | 4 | 4 | 4 | 4 | 0 |
| Ga | 2 | 2 | 4 | 3 | 5 | 4 | 4 | 5 | 4 | 3 | 2 | 2 |
| Xs | 2 | 2 | 4 | 2 | 5 | 4 | 4 | 5 | 5 | 3 | 2 | 3 |
| Ab | 2 | 2 | 4 | 3 | 4 | 4 | 4 | 4 | 3 | 2 | 1 | 2 |
| Co | 2 | 2 | 2 | 1 | 4 | 2 | 1 | 4 | 3 | 1 | 1 | 2 |
| Av | 0 | 0 | 4 | 3 | 5 | 4 | 4 | 5 | 4 | 3 | 2 | 0 |
| Dg | 2 | 0 | 2 | 0 | 5 | 5 | 4 | 4 | 4 | 3 | 0 | 1 |
| Al | 0 | 0 | 3 | 1 | 4 | 4 | 3 | 4 | 4 | 3 | 2 | 0 |
| St | 0 | 1 | 3 | 0 | 5 | 4 | 4 | 3 | 2 | 1 | 0 | 1 |
| Ec | 2 | 1 | 4 | 3 | 5 | 5 | 5 | 4 | 4 | 4 | 0 | 1 |
| Sh | 2 | 2 | 4 | 3 | 5 | 4 | 4 | 4 | 4 | 2 | 1 | 1 |
| Ag | 2 | 2 | 3 | 1 | 4 | 3 | 3 | 2 | 2 | 2 | 1 | 1 |
| Cn | 0 | 0 | 2 | 1 | 3 | 3 | 1 | 3 | 2 | 1 | 0 | 0 |

TABLE 3 (Continued)

Post-Emergent Herbicidal Activity

| Compound No. | 61 | 61 | 62 | 62 | 73 | 73 | 82 | 82 | 82 | 83 | 83 |
|---|---|---|---|---|---|---|---|---|---|---|---|
| Rate g/ha | 125 | 25 | 625 | 125 | 125 | 25 | 125 | 25 | 5 | 125 | 25 |
| Test Plants | | | | | | | | | | | |
| Sb | 4 | 3 | 5 | 5 | 4 | 3 | 3 | 3 | 0 | 3 | 3 |
| Rp | 4 | 3 | 5 | 4 | 4 | 2 | 4 | 4 | 3 | 4 | 3 |
| Ct | 2 | 1 | 4 | 1 | 2 | 2 | 2 | 1 | 0 | 2 | 0 |
| Sy | 4 | 3 | 5 | 5 | 3 | 3 | 3 | 2 | 1 | 3 | 3 |
| Mz | 4 | 2 | 5 | 5 | 4 | 2 | 4 | 4 | 3 | 4 | 4 |
| Ww | 4 | 3 | 4 | 4 | 1 | 1 | 3 | 2 | 3 | 3 | 3 |
| Rc | 2 | 1 | 5 | 2 | 2 | 2 | 1 | 0 | 0 | 2 | 2 |
| Bd | 4 | 4 | 5 | 5 | 4 | 3 | 4 | 2 | 3 | 4 | 3 |
| Ip | 4 | 3 | 4 | 4 | 4 | 2 | 3 | 3 | 2 | 3 | 2 |
| Am | 4 | 4 | 5 | 5 | 4 | 3 | 4 | 3 | 3 | 3 | 2 |
| Pi | 1 | 1 | 4 | 3 | 2 | 1 | 3 | 3 | 3 | 3 | 3 |
| Ca | 4 | 2 | 5 | 5 | 4 | 3 | 3 | 3 | 3 | 3 | 2 |
| Ga | 4 | 1 | 5 | 5 | 3 | 0 | 3 | 3 | 1 | 3 | 1 |
| Xs | 4 | 3 | 5 | 5 | 3 | 2 | 4 | 3 | 3 | 4 | 3 |
| Ab | 4 | 2 | 4 | 4 | 4 | 1 | 3 | 3 | 3 | 3 | 3 |
| Co | 3 | 2 | 5 | 5 | 3 | 1 | 3 | 1 | 1 | 1 | 0 |
| Av | 4 | 1 | 5 | 5 | 2 | 0 | 3 | 3 | 3 | 3 | 3 |
| Dg | 3 | 1 | 5 | 5 | 1 | 0 | 4 | 3 | 2 | 4 | 3 |
| Al | 3 | 2 | 4 | 4 | 3 | 2 | 3 | 3 | 2 | 1 | 0 |
| St | 4 | 3 | 5 | 5 | 3 | 1 | 4 | 2 | 1 | 4 | 3 |
| Ec | 5 | 2 | 5 | 5 | 2 | 1 | 4 | 3 | 3 | 5 | 4 |
| Sh | 4 | 2 | 5 | 5 | 3 | 1 | 4 | 3 | 3 | 4 | 4 |
| Ag | 3 | 1 | 4 | 4 | 1 | 1 | 3 | 3 | 2 | 3 | 2 |
| Cn | 2 | 0 | 2 | 2 | 1 | 0 | 3 | 3 | 1 | 3 | 3 |

TABLE 4

| Post-Emergent Herbicidal Activity | | | |
|---|---|---|---|
| Compound No. | 34 | 34 | 34 |
| Rate g/ha | 400 | 100 | 25 |
| Test Plants | | | |
| Sb | 9 | 8 | 8 |
| Rp | 9 | 8 | 5 |
| Ct | 5 | 3 | 0 |
| Sy | 9 | 8 | 8 |
| Mz | 8 | 7 | 4 |
| Ww | 7 | 5 | 3 |
| Rc | 3 | 3 | 0 |
| Bd | 9 | 8 | 6 |
| Ip | 6 | 5 | 5 |
| Am | 9 | 8 | 7 |
| Pi | 7 | 5 | 3 |
| Ca | 8 | 5 | 4 |
| Ga | 8 | 6 | 2 |
| Xs | 9 | 8 | 5 |
| Ab | 7 | 5 | 3 |
| Eh | 5 | 5 | 3 |
| Av | 6 | 5 | 5 |
| Dg | 8 | 9 | 2 |
| Al | 7 | 5 | 0 |
| St | 8 | 6 | 5 |
| Ec | 9 | 7 | 6 |
| Sh | 9 | 9 | 8 |
| Ag | 8 | 7 | 6 |
| Ce | 7 | 6 | 5 |

TABLE 5

Pre-Emergent Herbicidal Activity

| Compound No. | 2 | 2 | 2 | 19 | 19 | 29 | 30 | 30 | 31 | 31 |
|---|---|---|---|---|---|---|---|---|---|---|
| Rate g/ha | 625 | 125 | 25 | 625 | 125 | 125 | 125 | 25 | 625 | 125 |
| Test Plants | | | | | | | | | | |
| Sb | 5 | 4 | 1 | 4 | 3 | 0 | 4 | 2 | 5 | 4 |
| Rp | 5 | 5 | 4 | 4 | 4 | 4 | 4 | 2 | 5 | 4 |
| Ct | 4 | 3 | 1 | 3 | 2 | 2 | 3 | 2 | 4 | 2 |
| Sy | 4 | 4 | 4 | 3 | 2 | 0 | 4 | 3 | 4 | 4 |
| Mz | 5 | 4 | 3 | 5 | 5 | 1 | 3 | 2 | 4 | 4 |
| Ww | 5 | 5 | 3 | 5 | 5 | 2 | 5 | 2 | 5 | 4 |
| Rc | 5 | 4 | 0 | 5 | 0 | 1 | - | - | 5 | 5 |
| Bd | 4 | 3 | 0 | 4 | 2 | 4 | 4 | 2 | 4 | 1 |
| Ip | 3 | 2 | 0 | 2 | 2 | 2 | 3 | 1 | 4 | 3 |
| Am | 4 | 4 | 3 | 4 | 4 | 3 | 4 | 2 | 4 | 3 |
| Pi | 4 | 2 | 0 | 2 | 0 | 2 | 2 | 1 | 4 | 0 |
| Ca | 4 | 4 | 3 | 4 | 4 | 3 | 3 | 2 | 4 | 1 |
| Ga | 4 | 4 | 4 | 4 | 2 | - | 4 | 3 | 4 | 4 |
| Xa | 4 | 4 | 1 | 3 | 3 | 4 | 4 | 2 | 4 | 3 |
| Ab | 4 | 4 | 0 | 3 | 2 | 2 | 2 | 2 | 4 | 1 |
| Co | 0 | 0 | 2 | 1 | 0 | 2 | 2 | 2 | 1 | 0 |
| Av | 4 | 4 | 2 | 5 | 3 | 1 | 2 | 1 | 4 | 1 |
| Dg | 4 | 4 | 2 | 4 | 3 | 0 | 3 | 2 | 4 | 4 |
| Al | 4 | 4 | 0 | 4 | 3 | 3 | 4 | 2 | 5 | 3 |
| St | 5 | 4 | 4 | 5 | 3 | 1 | 2 | 1 | 5 | 4 |
| Ec | 5 | 4 | 3 | 5 | 4 | 0 | 2 | 0 | 5 | 3 |
| Sh | 4 | 4 | 4 | 4 | 4 | 2 | 2 | 0 | 5 | 4 |
| Cn | 5 | 5 | 4 | 5 | 3 | 0 | 2 | 0 | 5 | 3 |

TABLE 5 (Continued)

Pre-Emergent Herbicidal Activity

| Compound No. | 33 | 33 | 39 | 39 | 46 | 47 | 49 | 49 | 55 | 55 |
|---|---|---|---|---|---|---|---|---|---|---|
| Rate g/ha | 625 | 125 | 625 | 125 | 125 | 125 | 625 | 125 | 625 | 125 |
| Test Plants | | | | | | | | | | |
| Sb | 4 | 4 | 4 | 4 | 3 | 4 | 4 | 2 | 4 | 3 |
| Rp | 5 | 4 | 4 | 4 | 4 | 4 | 4 | 3 | 4 | 4 |
| Ct | 4 | 1 | 3 | 3 | 4 | 4 | 4 | 4 | 4 | 3 |
| Sy | 4 | 4 | 4 | 4 | 4 | 4 | 4 | 4 | 4 | 4 |
| Mz | 4 | 2 | 4 | 4 | 2 | 4 | 4 | 3 | 4 | 2 |
| Ww | 4 | 4 | 5 | 4 | 4 | 5 | 5 | 4 | 4 | 0 |
| Rc | 4 | 4 | 4 | 0 | 4 | 5 | 5 | 5 | 2 | 0 |
| Bd | 5 | 5 | 4 | 1 | 4 | 4 | 4 | 4 | 4 | 4 |
| Ip | 4 | 2 | 4 | 1 | 3 | 4 | 3 | 2 | 4 | 4 |
| Am | 5 | 4 | 4 | 2 | 4 | 4 | 4 | 4 | 4 | 4 |
| Pi | 2 | 2 | 3 | 0 | 1 | 0 | 1 | 0 | 4 | 1 |
| Ca | 4 | 4 | 3 | 0 | 4 | 4 | 4 | 3 | 4 | 4 |
| Ga | 4 | 4 | 4 | 4 | - | 4 | 4 | 3 | 4 | 4 |
| Xa | 4 | 2 | 3 | 0 | 4 | 4 | 4 | 3 | 4 | 2 |
| Ab | 3 | 1 | 1 | 0 | 3 | 3 | 4 | 2 | 4 | 1 |
| Co | 1 | 0 | 0 | 0 | 2 | 0 | 2 | 3 | 2 | 1 |
| Av | 4 | 3 | 4 | 3 | 3 | 4 | 4 | 3 | 4 | 2 |
| Dg | 4 | 4 | 5 | 4 | 2 | 4 | 4 | 3 | 4 | 3 |
| Al | 4 | 3 | 4 | 4 | 4 | 4 | 4 | 4 | 4 | 3 |
| St | 5 | 4 | 4 | 2 | 4 | 5 | 5 | 5 | 4 | 4 |
| Ec | 4 | 3 | 5 | 3 | 3 | 5 | 4 | 3 | 4 | 4 |
| Sh | 4 | 3 | 1 | 0 | 1 | 4 | 4 | 3 | 4 | 0 |
| Cn | 4 | 0 | 4 | 1 | 3 | 4 | 4 | 0 | 4 | 0 |

TABLE 5 (Continued)

Pre-Emergent Herbicidal Activity

| Compound No. | 61 | 62 | 62 | 73 | 82 | 82 | 83 | 83 |
|---|---|---|---|---|---|---|---|---|
| Rate g/ha | 125 | 625 | 125 | 125 | 625 | 125 | 625 | 125 |
| Test Plants | | | | | | | | |
| Sb | 4 | 4 | 2 | 3 | 3 | 0 | 3 | 2 |
| Rp | 4 | 5 | 4 | 3 | 4 | 4 | 4 | 2 |
| Ct | 4 | 4 | 0 | 2 | 3 | 1 | 2 | 0 |
| Sy | 4 | 4 | 4 | 4 | 4 | 3 | 3 | 1 |
| Mz | 4 | 4 | 3 | 3 | 5 | 4 | 5 | 4 |
| Ww | 5 | 5 | 5 | 4 | 5 | 5 | 5 | 5 |
| Rc | 5 | 5 | 5 | 5 | 5 | 3 | 5 | 3 |
| Bd | 4 | 4 | 4 | 3 | 4 | 3 | 4 | 4 |
| Ip | 4 | 4 | 1 | 2 | 3 | 1 | 2 | 2 |
| Am | 4 | 4 | 4 | 3 | 4 | 4 | 4 | 4 |
| Pi | 1 | 0 | 0 | 0 | 4 | 0 | 3 | 0 |
| Ca | 4 | 4 | 4 | 4 | 4 | 3 | 4 | 3 |
| Ga | - | 4 | 3 | 2 | 4 | 3 | 4 | - |
| Xa | 4 | 4 | 4 | 2 | 4 | 4 | 4 | 2 |
| Ab | 3 | 4 | 1 | 1 | 3 | 2 | 3 | 2 |
| Co | 2 | 1 | 0 | 1 | 0 | 0 | 0 | 0 |
| Av | 4 | 4 | 1 | 1 | 4 | 1 | 4 | 2 |
| Dg | 3 | 5 | 3 | 0 | 5 | 4 | 4 | 3 |
| Al | 4 | 5 | 3 | 3 | 4 | 2 | 4 | 3 |
| St | 5 | 5 | 4 | 3 | 3 | 2 | 4 | 3 |
| Ec | 3 | 5 | 4 | 1 | 4 | 3 | 5 | 5 |
| Sh | 4 | 5 | 4 | 3 | 4 | 3 | 4 | 4 |
| Cn | 4 | 4 | 4 | 1 | 5 | 3 | 5 | 4 |

## Claims

1. A compound of the formula I and the salts thereof

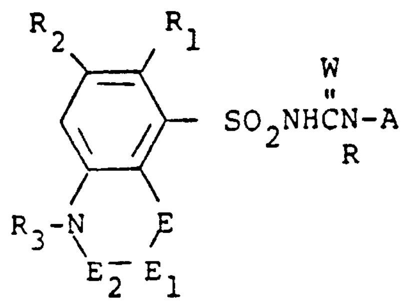

I

wherein
W is O or S;
E is O, $S(O)_m{}'$ or $N$-$R_4$;
m is 0, 1 or 2;
$E_1$ and $E_2$ are independently $CH_2$, $CH_2CH_2$, $CH(C_1$-$C_4$ alkyl), $C(C_1$-$C_4$ alkyl$)_2$ or CH (aryl), with the proviso that $E_1$ and $E_2$ cannot both be $CH_2CH_2$;
$R_1$ and $R_2$ are independently hydrogen, halogen, $C_1$-$C_4$ alkyl, $C_1$-$C_4$ haloalkyl, arylalkyl, nitro, $C_1$-$C_4$ alkoxy, $C_1$-$C_4$ haloalkoxy, cyano, carboxy, carbamoyl, N-($C_1$-$C_4$ alkyl)carbamoyl, N,N-di($C_1$-$C_4$ alkyl)carbamoyl, $C_1$-$C_4$ alkoxycarbonyl, $C_1$-$C_4$ alkylthio, $C_1$-$C_4$ alkylsulfinyl, $C_1$-$C_4$ alkylsulfonyl, sulfamoyl, $C_1$-$C_4$ alkylsulfamoyl, di($C_1$-$C_4$ alkyl) sulfamoyl, amino, $C_1$-$C_4$ alkylamino or di($C_1$-$C_4$ alkyl) amino, $C_1$-$C_4$ acylamino;
R is selected from hydrogen, optionally substituted $C_1$-$C_4$ alkyl, $C_2$-$C_4$ alkenyl, $C_2$-$C_4$ alkynyl, or substituted aryl or arylalkyl;
$R_3$ and $R_4$ are each independently selected from hydrogen, optionally substituted $C_1$-$C_4$ alkyl, $C_2$-$C_4$ alkenyl, $C_2$-$C_4$ alkynyl, optionally substituted aryl or arylalkyl, or $C_1$-$C_4$ acyl;

A is 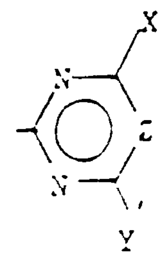 , 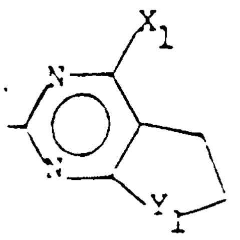 , 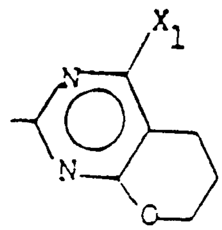 , 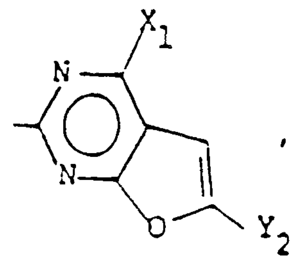 ,

A-1 A-2 A-3 A-4

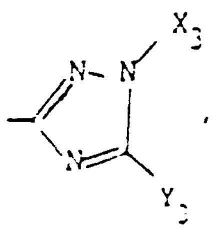 , 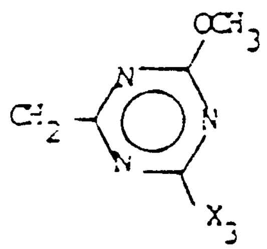 or 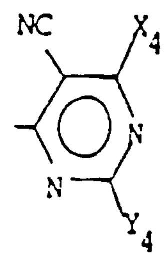

A-5 A-6 A-7

X is $CH_3$, $OCH_3$, $OCH_2CH_3$, Cl, F, Br, I, $OCF_2H$, $CH_2F$ or $CF_3$,
Y is H, $CH_3$, $OCH_3$, $OC_2H_5$, $CH_2$ $OCH_3$, $NH_2$, $NHCH_3$, $N(CH_3)_2$, $C_2H_5$, $CF_3$, $SCH_3$, $N(OCH_3)CH_3$, $OCH_2CH$-$CH_2$, $OCH_2C$-CH, $OCH_2CF_3$, cyclopropyl, $OCH_2CH_2OCH_3$, $CH_2SCH_3$, $(C=O)R_5$, $CR_5(OCH_3)_2$, 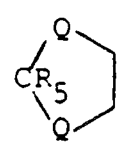

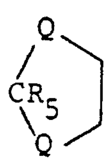 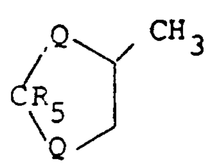 ,

$CR_5(OCH_2CH_3)_2$, $OCF_2H$, $SCF_2H$, C = CH or C = $CCH_3$ where Q is O or S and $R_5$ is H or $CH_3$; Z is CH, N, $CCH_3$, $CC_2H_5$, CCl or CBr; $Y_1$ is O or $CH_2$; $X_1$ is $CH_3$, $OCH_3$, $OC_2H_5$ or $OCF_2H$; $Y_2$ is H or $CH_3$; $X_2$ is $CH_3$, $C_2H_5$ or $CH_2CF_3$; $Y_3$ is $OCH_3$, $OC_2H_5$, $SCH_3$, $SC_2H_5$, $CH_3$ or $CH_2CH_3$; $X_3$ is $CH_3$ or $OCH_3$; $Y_4$ is $CH_3$, $OCH_3$, $OCH_2CH_3$ or Cl; and $X_4$ is $CH_3$, $OCH_3$, $OCH_2CH_3$, $CH_2OCH_3$ or Cl.

2. A compound according to claim 1, wherein $R_3$ and $R_4$ are independently selected from hydrogen, optionally substituted $C_1$-$C_4$ alkyl, $C_2$-$C_4$ alkenyl, $C_2$-$C_4$ alkynyl or optionally substituted aryl or arylalkyl.

3. A compound according to claim 1 or claim 2, wherein $E_1$ is selected from $CH_2$, $CH_2CH_2$, $CH(C_1$-$C_4$ alkyl) or $C(CH_3)_2$.

4. A compound according to claim 1 or claim 2, wherein $E_2$ is $CH_2$ and A is A-1.

5. A compound according to claim 1 or claim 2, wherein R is hydrogen; $R_1$ is hydrogen, fluorine, chlorine, bromine, methyl, methoxy, trifluoromethyl methoxycarbonyl, N,N-dimethyl-carbamoyl, or methylthio; and Y is methyl, methoxy, ethoxy, methoxymethyl, trifluoromethyl, 2,2,2-trifluoroethoxy, dimethoxymethyl, difluoromethoxy or methylamino.

6. A compound according to claim 1 or claim 2, wherein: E is O, S, NH or $NCH_3$; $E_1$ is $CH_2$, $CH_2CH_2$ OR $CH(CH_3)$; $E_2$ is $CH_2$ and $R_3$ is hydrogen, methyl or ethyl.

7. A compound of formula II

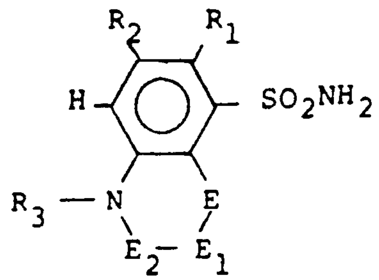

II

wherein $R_1$, $R_2$, E, $E_1$ and $E_2$ are as defined in claim 1.

8. A compound of formula II

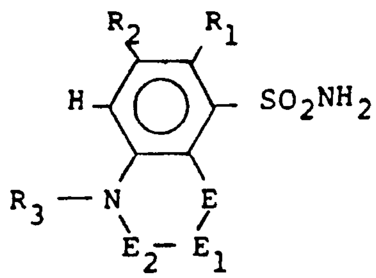

II

wherein $R_1$, $R_2$, E, $E_1$ and $E_2$ are as defined in claim 2.

9. A process for the preparation of a compound according to claim 1 or claim 2 by the reaction of sulfonylisocyanate or a sulfonylisothiocyanate of formula X with a heterocyclic amine of formula IX.

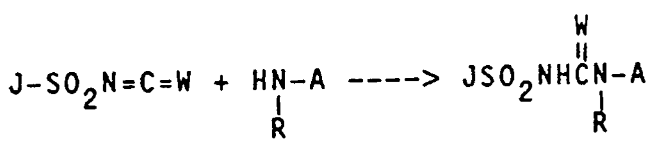

VIII VII I

where J represents the bicyclic system

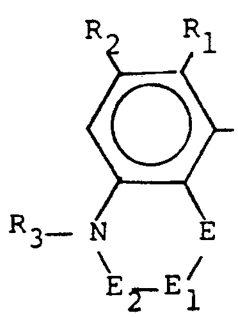

and W, A and R are as defined in claim 1 or claim 2.

10. A process for the preparation of a compound according to claim 1 or claim 2, where W is oxygen, by the reaction of a phenyl carbamate of formula XI with an amine of formula IX.

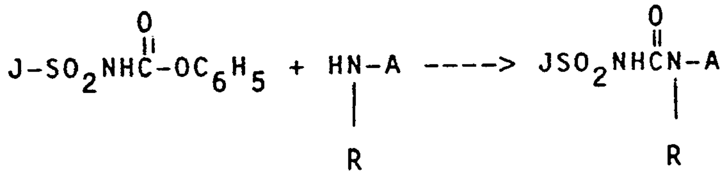

IX VII Ia

wherein A and J are as defined in claim 7 or claim 8.

11. A process for the preparation of a compound according to claim 1 or claim 2, where W is oxygen by the reaction of a heterocyclic carbamate of formula XII with a sulfonamide of formula II.

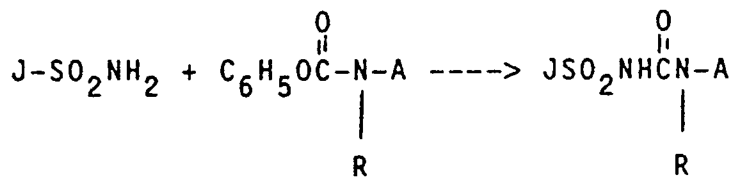

II X Ia

wherein A and J are as defined in claim 7 or claim 8.

12. A process for the preparation of a compound according to claim 1 or claim 2, where R is hydrogen, by the reaction of a heterocyclic isocyanate or isothiocyanate of formula XI with a sulfonamide of formula II

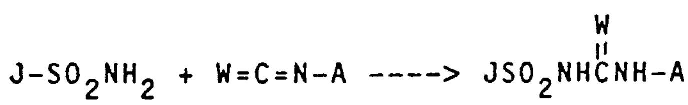

II XI 1b

wherein A and J are as defined in claim 7 or claim 8.

13. A process for the preparation of a compound according to claim 1 or claim 2, where A is hydrogen and W is oxygen by the reaction of a sulfonamide of formula II with a heterocyclic amide of formula XII.

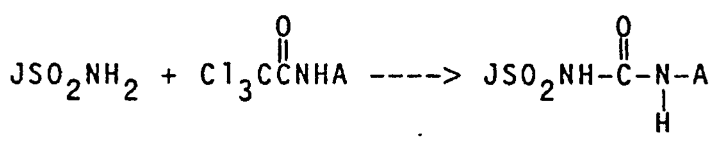

wherein A and J are as defined in claim 7 or 8.

14. A herbicidal composition comprising a compound according to claim 1 or claim 2.

15. A process for severely damaging or killing unwanted plants which process comprises applying to the plants, or to the growth medium of the plants, an effective amount of a compound according to claim 1 or claim 2.

European Patent Office

# EUROPEAN SEARCH REPORT

Application Number

EP 90 30 3806

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| A | EP-A-0 082 681 (E.I. DUPONT DE NEMOURS AND CO.)<br>* claims 1,31-33 *<br>--- | 1,7-15 | C 07 D 417/12<br>C 07 D 413/12<br>C 07 D 403/12<br>C 07 D 491/04<br>C 07 D 279/16<br>C 07 D 265/36<br>C 07 D 241/42<br>A 01 N 47/36 //<br>(C 07 D 491/04<br>C 07 D 307:00<br>C 07 D 239:00 )<br>(C 07 D 491/04<br>C 07 D 311:00<br>C 07 D 239:00 ) |
| A | EP-A-0 099 339 (CIBA-GEIGY AG)<br>* claims 1,21,25-34 *<br>--- | 1,7-15 | |
| A | EP-A-0 155 767 (E.I. DUPONT DE NEMOURS AND CO.)<br>* claims 1,13-16 *<br>--- | 1,7-15 | |
| A | EP-A-0 166 516 (E.I. DUPONT DE NEMOURS AND CO.)<br>* claims 1,37-40 *<br>----- | 1-7-15 | |

TECHNICAL FIELDS SEARCHED (Int. Cl.5)

C 07 D 417/00
C 07 D 413/00
C 07 D 403/00
C 07 D 491/00
C 07 D 279/00
C 07 D 265/00
C 07 D 241/00
A 01 N 47/00

The present search report has been drawn up for all claims

| Place of search | Date of completion of the search | Examiner |
|---|---|---|
| BERLIN | 01-08-1990 | VAN AMSTERDAM L.J.P. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)